# EUROPEAN PATENT SPECIFICATION

(11) **EP 3 813 869 B1**
(45) Date of publication and mention of the grant of the patent: **18.10.2023**
(21) Application number: 19746140.3
(22) Date of filing: 24.05.2019
(51) Int. Cl.: A61K 38/18, A61P 25/16, A61P 25/28

(54) **CCR5 INHIBITOR FOR USE IN TREATING A NEUROINFLAMMATORY DISORDER THAT INVOLVES CEREBRAL INFLAMMATION**
CCR5-INHIBITOR ZUR VERWENDUNG BEI DER BEHANDLUNG EINER NEUROINFLAMMATORISCHEN ERKRANKUNG, DIE EINE GEHIRNENTZÜNDUNG BEINHALTET
INHIBITEUR DU CCR5 POUR LE TRAITEMENT D'UN TROUBLE NEUROINFLAMMATOIRE IMPLIQUANT UNE INFLAMMATION CÉRÉBRALE

(30) Priority: 28.05.2018 US 201862677113 P
(43) Date of publication of application: 05.05.2021
(73) Proprietor: Université de Genève, 1211 Genève (CH); Orion Biotechnology Switzerland Sàrl, 1202 Geneva (CH); Hopitaux Universitaires de Geneve, 1205 Geneva (CH)
(72) Inventor: MERKLER, Doron, 1206 Geneva (CH); STEINBACH, Karin Elke, 1201 Geneva (CH); HARTLEY, Oliver, 1256 Troinex (CH)
(74) Representative: E. Blum & Co. AG
(86) International application number: PCT/IB2019/054349
(87) International publication number: WO 2019/229615

(56) References cited:
- US-B2- 8 686 111
- MASARU MATSUI ET AL: "Treatment of experimental autoimmune encephalomyelitis with the chemokine receptor antagonist Met-RANTES", JOURNAL OF NEUROIMMUNOLOGY., vol. 128, no. 1-2, 1 July 2002 (2002-07-01), pages 16-22, XP055625974, NL ISSN: 0165-5728, DOI: 10.1016/S0165-5728(02)00121-2
- MÁRCIA CARVALHO VILELA ET AL: "The Chemokine CCL5 Is Essential for Leukocyte Recruitment in a Model of Severe Herpes simplex Encephalitis", ANNALS OF THE NEW YORK ACADEMY OF SCIENCES, vol. 1153, no. 1, 1 February 2009 (2009-02-01), pages 256-263, XP055626035, US ISSN: 0077-8923, DOI: 10.1111/j.1749-6632.2008.03959.x
- JIIN-TARNG LIOU ET AL: "Peritoneal Administration of Met-RANTES Attenuates Inflammatory and Nociceptive Responses in a Murine Neuropathic Pain Model", JOURNAL OF PAIN, vol. 14, no. 1, 1 January 2013 (2013-01-01), pages 24-35, XP055625961, US ISSN: 1526-5900, DOI: 10.1016/j.jpain.2012.09.015
- ANNA MARIA PAPINI ET AL: "Novel diagnostic tools and solutions for multiple sclerosis treatment: a patent review (2009 - 2014)", EXPERT OPINION ON THERAPEUTIC PATENTS, vol. 25, no. 8, 8 May 2015 (2015-05-08), pages 873-884, XP055626055, ISSN: 1354-3776, DOI: 10.1517/13543776.2015.1043267

## Description

### CROSS-REFERENCE TO RELATED APPLICATIONS

This application claims the benefit of and priority from United States Provisional Patent Application No. 62/677,113 filed on May 28, 2018.

### FIELD

The present application relates to an inhibitor inhibiting cerebral inflammation for use in treating a neuroinflammatory disorder.

### BACKGROUND

Chemokine and chemokine receptors have been implicated in a wide range of inflammatory diseases, including neuroinflammatory diseases that are characterized by inflammation in the central nervous system (CNS). One chemokine can bind to several chemokine receptors and vice versa, so that they form a redundant system.

Among the known chemokine receptors, C-C chemokine receptor type 5 (CCR5) has been identified as a co-receptor that mediates HIV entry. Maraviroc, a CCR5 inhibitor, has been approved for treatment of HIV. The C-C chemokines CCL3, CCL4 and CCL5 are the main CCR5 endogenous agonists. CCL5, also known as RANTES (regulated on activation, normal T cell expressed and secreted), has been shown to interact with C-C chemokine receptor type 1 (CCR1) and C-C chemokine receptor type 3 (CCR3) in addition to CCR5.

CCR5 inhibitors have been tested in murine Experimental Autoimmune Encephalomyelitis (EAE) models, which are commonly used animal models of human CNS demyelinating diseases, including multiple sclerosis (MS) and acute disseminated encephalomyelitis (ADEM).

In existing EAE models, neuro inflammation and tissue destruction are elicited predominantly in the spinal cord, thereby providing an incomplete representation of human MS pathology, which takes place mainly in the brain and rarely affects the spinal cord only.

US 8,686,111 relates to fully coded polypeptide derivatives of a CCR5 inhibitor (RANTES) and uses thereof for the treatment of HIV infections. Said polypeptide derivatives comprise an N-terminal portion and a C-terminal portion, wherein said N-terminal portion comprises the signature sequence QGP[P or L] and the amino acid sequence of said C-terminal portion is at least 70% identical to SEQ ID NO: 1.

Jiin-Tarng Liou et al. ("Peritoneal Administration of Met-RANTES Attenuates Inflammatory and Nociceptive Responses in a Murine Neuropathic Pain Model", Journal of Pain, vol. 14, no. 1, 2013-01-01) describes that CCR5 inhibitor Met-RANTES reduces inflammatory responses in a neuropathic pain model using mice having a partial sciatic nerve ligation.

Anna Maria Papini et 1. ("Novel diagnostic tools and solutions for multiple sclerosis treatment: a patent review (2009 - 2014)", Expert Opinon on Therapeutic Patents, vol. 25, no. 8, 2015-05-08) reviews therapies against multiple sclerosis.

### SUMMARY

The invention is defined in the appended claims. In one aspect, there is provided a CCR5 inhibitor for use in a method of treating a neuroinflammatory disorder that involves cerebral inflammation in a subject. The CCR5 inhibitor for use in said medical method as described herein is a polypeptide comprising an N-terminal portion and a C-terminal portion, wherein the N-terminal portion comprises the signature sequence QGP[P or L] and the amino acid sequence of the C-terminal portion is at least 70% identical to SEQ ID NO: 1.

In an embodiment of the method as described herein, the CCR5 inhibitor leads to a signaling response of 30% or less of the maximum response (Emax) elicited by PSC-RANTES, when tested at a concentration of 300 nM in a Calcium Flux signaling assay.

In an embodiment of the method as described herein, the CCR5 inhibitor is selective for CCR5 over CCR1 and CCR3.

In an embodiment of the method as described herein, the signature sequence is QGP[P or L] [L or G or S or M] [M or D or S or Q or G].

In an embodiment of the method as described herein, the signature sequence is QGP[P or L][L or G or S or M][M or D or S or Q or G]XX[Q or G or L or A or T or S]X, wherein X denotes any natural or modified amino acid.

In an embodiment of the method as described herein, the signature sequence is QGP[P or L]LM or QGPPG[D or S].

In an embodiment of the method as described herein, the signature sequence is QGPPLM or QGPPGD.

In an embodiment of the method as described herein, the signature sequence is QGP[P or L][L or M][M or Q][A or W or G or Q or N]X[Q or G or L][S or V or T or G], wherein X denotes any natural or modified amino acid.

In an embodiment of the method as described herein, the signature sequence is QGPPLM[A or W][L or T or M][Q or G][S or V or T or G].

In an embodiment of the method as described herein, the signature sequence is QGPP[G or L][M or Q]XX[Q or S][S or V], wherein X denotes any natural or modified amino acid.

In an embodiment of the method as described herein, the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) and QGPPMQGGLS (SEQ ID NO: 43).

In an embodiment of the method as described herein, the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) and QGPPLMQTTP (SEQ ID NO: 15).

In an embodiment of the method as described herein, the signature sequence is QGPPLMATQS (SEQ ID NO: 9).

In an embodiment of the method as described herein, the polypeptide comprises the amino acid sequence of SEQ ID NO: 70.

According to the claimed invention the method of treating as described herein is applied for a subject who suffers from or is susceptible to a neuroinflammatory disorder that involves cerebral inflammation.

In an embodiment of the method as described herein, the neuroinflammatory disorder is Alzheimer's Disease, Parkinson's Disease, multiple sclerosis, viral encephalitis, Rasmussen encephalitis, progressive multifocal leukoencephalopathy

(PML) including PML-associated immune reconstitution inflammatory syndrome (IRIS), cerebral malaria, HIV-associated neurocognitive disorders, CNS vasculitis, antibody-mediated inflammatory brain disorders, or neurosarcoidosis.

In an embodiment of the method as described herein, the neuro inflammatory disease is multiple sclerosis, Rasmussen's encephalitis, progressive multifocal leukoencephalopathy (PML) including PML-associated immune reconstitution inflammatory syndrome (IRIS), cerebral malaria or HIV-associated neurocognitive disorders.

In an embodiment of the method as described herein, the neuro inflammatory disease is multiple sclerosis.

Other aspects and features of the present invention will become apparent to those of ordinary skill in the art upon review of the following description of specific embodiments of the invention in conjunction with the accompanying figures.

### BRIEF DESCRIPTION OF THE FIGURES

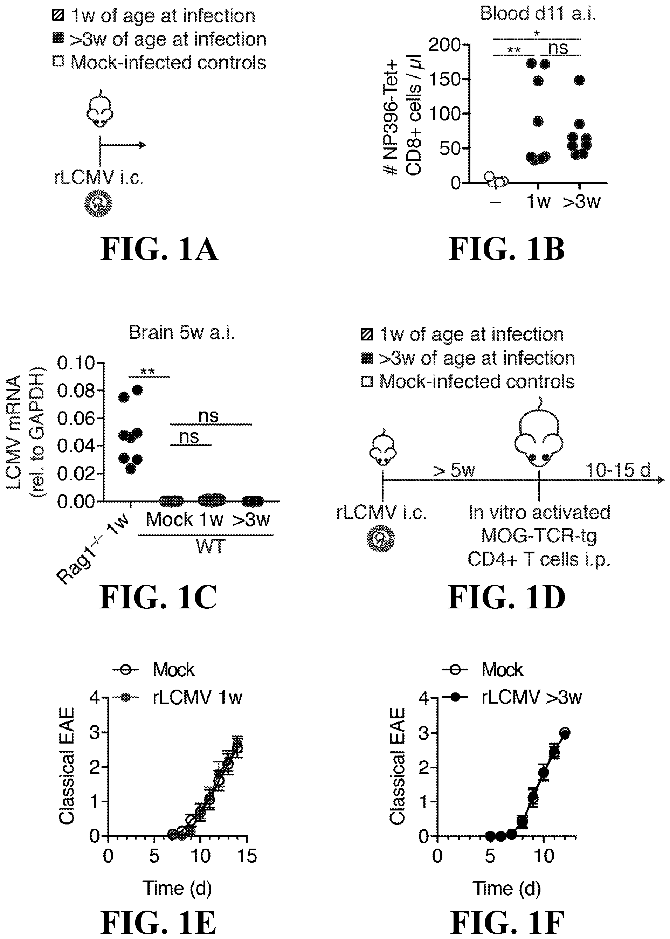

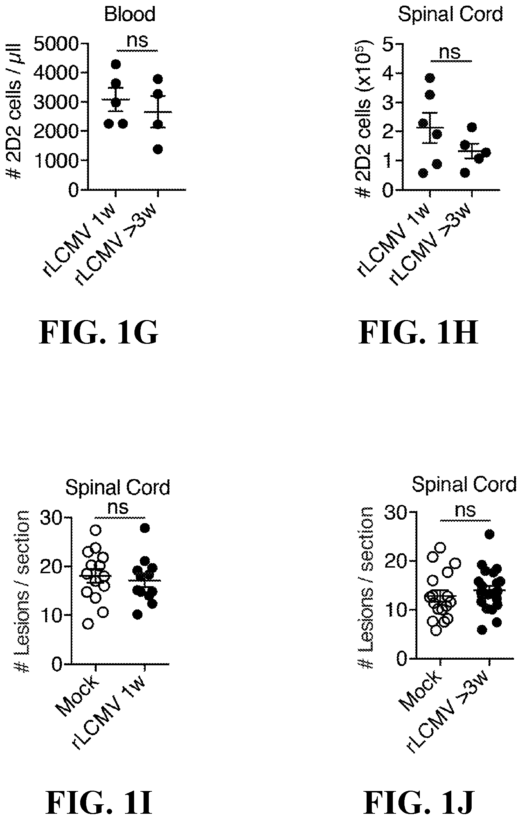

Figure 1 shows that transient intracerebral infection with rLCMV (lymphocytic choriomeningitis virus) does not influence CD4+ Tcell-driven spinal cord inflammation and classical EAE. (a) Experimental setup for b and c. (b) rLCMV-specific CD8+ T cell response after intracranial (i.c.) infection in 1w-old or >3w-old mice. (c) rLCMV clearance in brains 5w after i.c. infection in comparison to T cell-deficient Rag1^{-/-} mice. (d) Experimental setup for e-j. 1w-old or >3w-old WT mice were infected with rLCMV i.c.. >5w later, in vitro-primed 2D2 T cells were transferred, (e-f) Development of classical EAE after transfer of 2D2 T cells. Data represents mean ± s.e.m. (n = 12-23 mice per group). (g) Numbers of 2D2 T cells in peripheral blood two weeks after transfer. (h) 2D2 T cell infiltration into the spinal cord. (i-j) Histological analysis of spinal cord inflammation two weeks after 2D2 T cell transfer. Unless stated differently, symbols represent individual mice. Data is pooled (b, c, e, f, i, j) or representative (g, h) of two independent experiments. b-c: One-way ANOVA, g-j: Student's t-test.

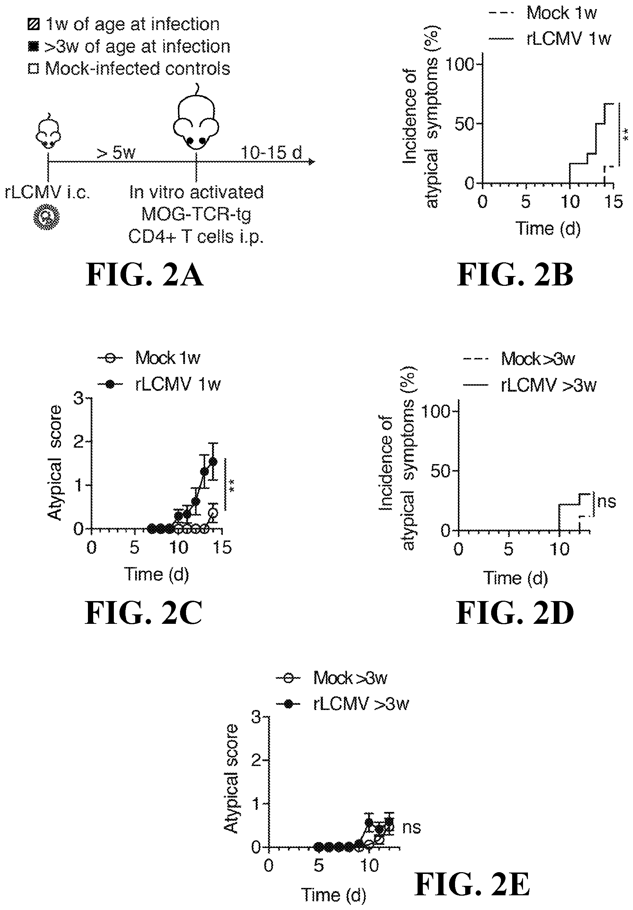

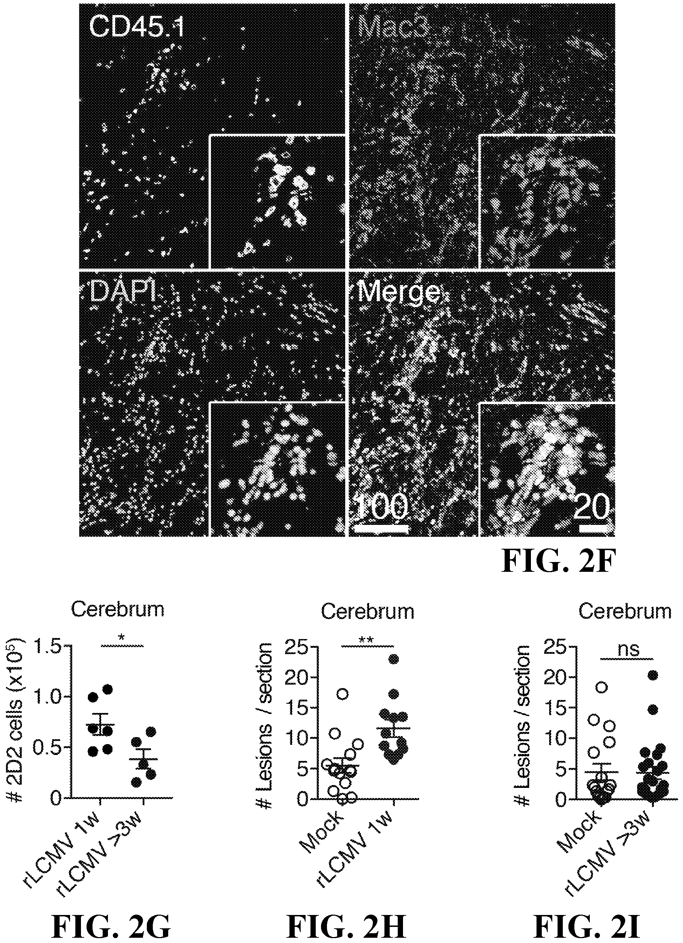

Figure 2 shows that transient intracerebral infection early in life predisposes to the development of brain inflammation by autoantigen-primed T cells. (a) Experimental setup: At 1w or >3w of age, wt mice were injected i.c. with rLCMV or vehicle, respectively. At least five weeks later, in vitro-primed 2D2 T cells were transferred intraperitoneally (i.p.) to induce EAE. (b) Incidence and (c) scores of atypical symptoms for mice infected at 1w of age in comparison to age-matched mock-infected controls. (d, e) as in (b, c), for mice infected >3w of age. (f) Representative image of a perivascular brain lesion after transfer of CD45.1+ 2D2 T cells into mice infected with rLCMV i.c. at 1w of age. Mac3 staining visualizes the recruitment of activated macrophages from the periphery, (g) 2D2 T cell infiltration into the cerebrum of mice infected at 1w or 3w of age. (h, i) Histological analysis of cerebral inflammation two weeks after 2D2 T cell transfer. Symbols represent individual mice, except for c, e where data represents mean ± s.e.m. (n = 12-23 mice per group). Data is pooled (b-e, h-i) or representative (g) of two independent experiments. b, d: Log-rank (Mantel-Cox) test, c, e: rm-two-way ANOVA, g-i: Student's t-test.

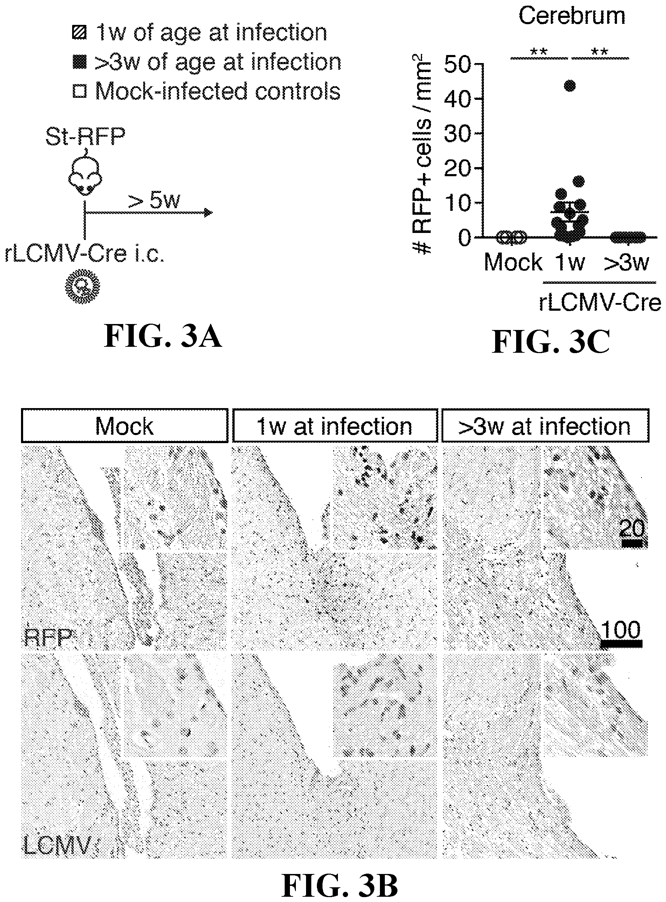

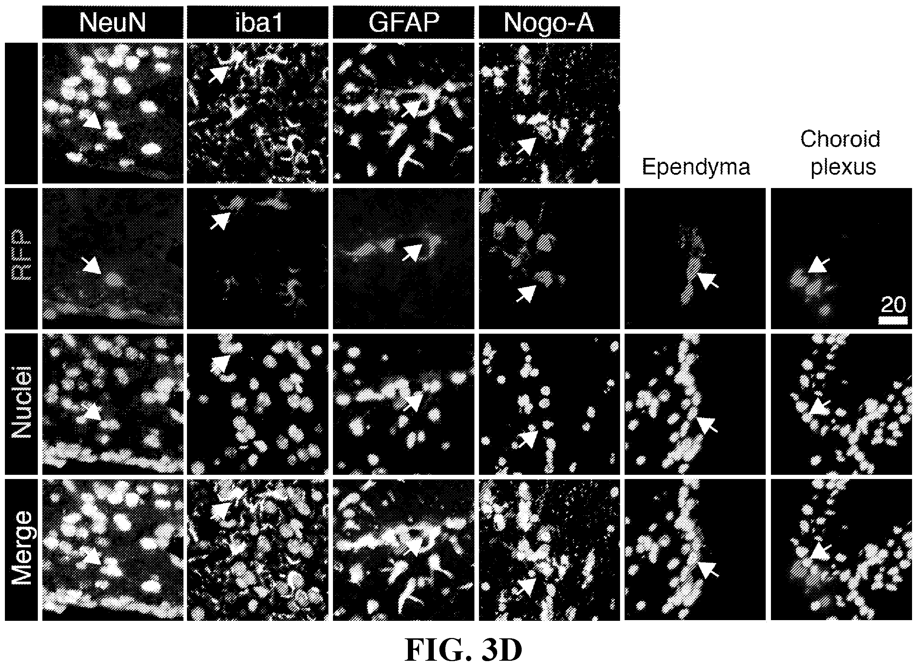

Figure 3 shows that non-cytolytic clearance in 1w old mice marks sites of previous infection. (a) Experimental setup: LoxP-flanked Stop-RFP reporter mice (St-RFP) were infected i.c. with rLCMV-Cre. (b) Representative images of RFP+ marker cells > 5w after infection compared to absent staining of LCMV (NP) in adjacent sections, (c) Quantification of RFP+ marker cells 5w after rLCMV-Cre i.c. infection. Symbols represent individual mice and mean ± s.e.m. are shown. Data represents a pool of two independent experiments (n = 4-16 mice per group). (d) RFP-expression was detected in neurons (NeuN+), microglia (iba1+), astrocytes (GFAP+) and oligodendrocytes (Nogo-A+) as well as in ependymal cells and choroid plexus cells at the ventricle. Representative images of n=3 mice are shown.

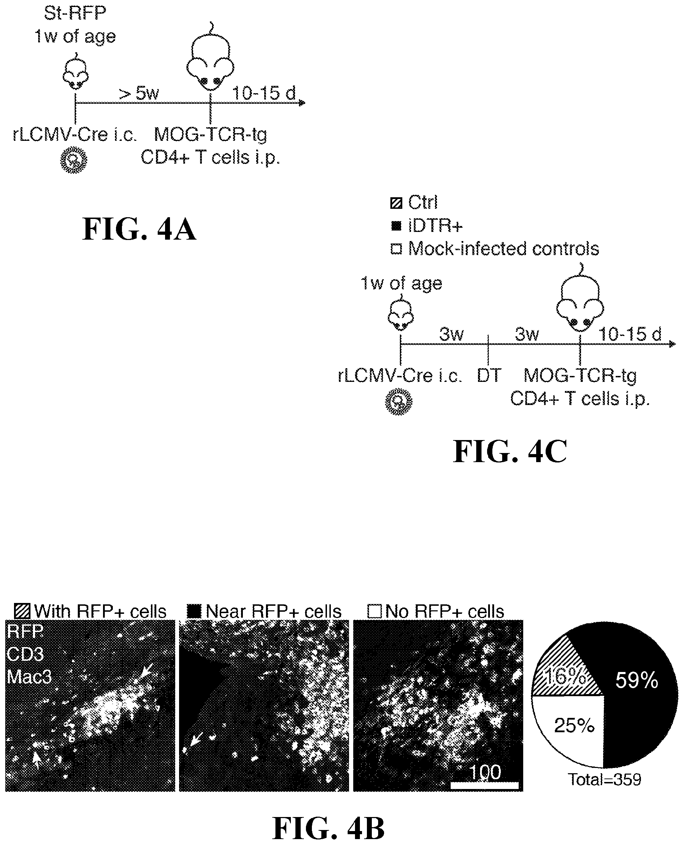

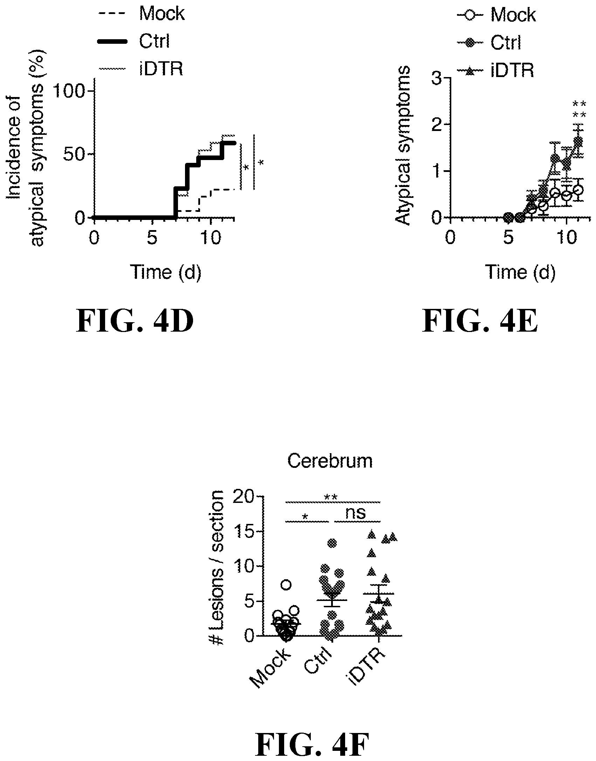

Figure 4 shows that autoimmune lesions in mice infected at 1w of age develop preferentially close to sites of resolved virus infection. (a) Experimental setup: LoxP-flanked Stop-RFP reporter mice (St-RFP) were infected i.e. with rLCMV-Cre and in vitro-primed 2D2 T cells were transferred >5w after infection. (b) Spatial correlation of autoimmune lesions containing T cells (CD3) and activated Macrophages (Mac3) with RFP+ marker cells two weeks after transfer of 2D2 T cells in mice infected at 1w of age. Data represents spatial analysis of a total of n=359 lesions (n =10 mice). (c) Cre recombinase-inducible diphtheria toxin receptor mice (iDTR) were infected i.c. with rLCMV-Cre at 1w of age. Virus-experienced cells were ablated by diphtheria toxin (DT) administration 3 weeks before transfer of 2D2 T cells, (d) Incidence and (e) scores of atypical symptoms of ablated iDTR mice in comparison to DT-treated infected wt littermates and mock-infected controls. Data in (e) represents mean ± s.e.m. (n=15-17 mice per group). (f) Histological analysis of cerebral inflammation two weeks after 2D2 T cell transfer. Symbols represent individual mice and mean ± s.e.m. are shown. All data are pooled from two independent experiments. d: Log-rank (Mantel-Cox) tests against mock-infected controls, e: rm-two-way ANOVA, f: one-way ANOVA.

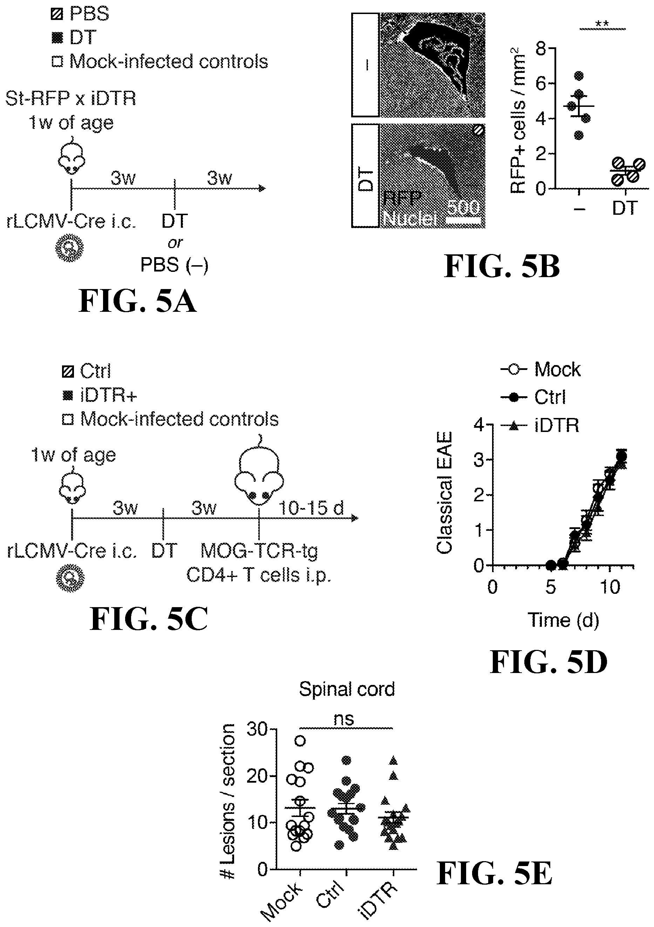

Figure 5 shows diphtheria toxin (DT)-mediated ablation of virus-experienced cells. (a) Experimental setup: LoxP-flanked Stop-RFP reporter mice (St-RFP) were crossed to Cre recombinase-inducible diphtheria toxin receptor mice (iDTR) and St-RFP x iDTR offspring was infected i.c. with rLCMV-Cre. After 3 weeks, mice were treated with DT or PBS (-). (b) Quantification of RFP+ virus-experienced cells 3 weeks after DT administration. One out of two independent experiments is shown. (c) Experimental setup for (d-e): Cre recombinase-inducible diphtheria toxin receptor mice (iDTR) were infected i.c. with rLCMV-Cre at 1w of age. Virus-experienced cells were ablated by diphtheria toxin (DT) administration 3 weeks before transfer of 2D2 T cells, (d) Development of classical EAE (mean ± s.e.m. for n=15-17 mice per group) and (e) spinal cord inflammation was analyzed two weeks after 2D2 T cell transfer in ablated iDTR mice in comparison to DT-treated infected wt littermates and mock-infected controls. Symbols represent individual mice and mean ± s.e.m. are shown. Data in (d-e) represents a pool of two independent experiments. b: Student's t-test, e: One-way ANOVA.

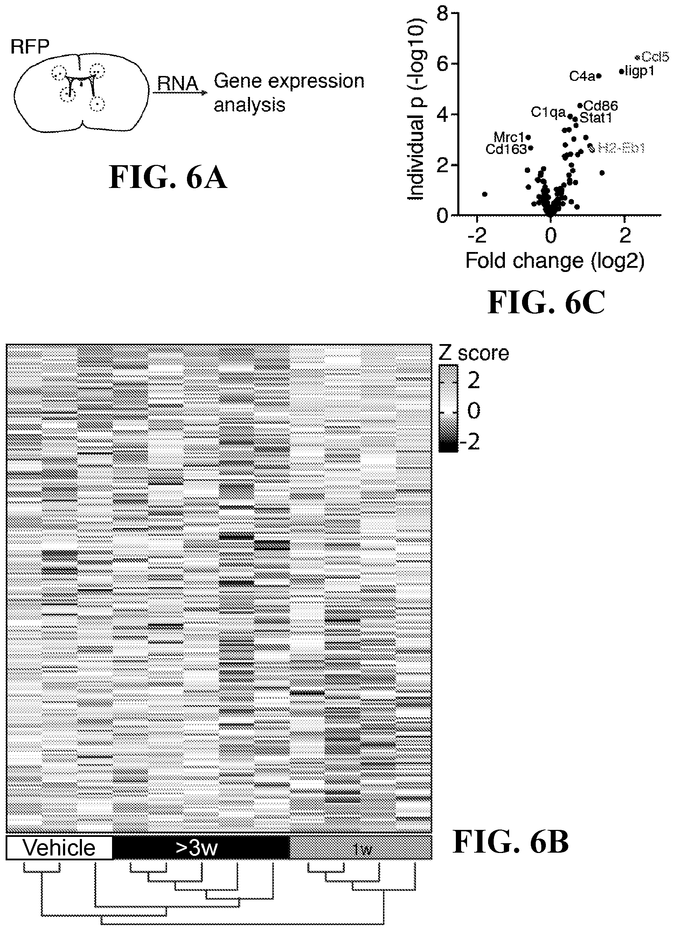

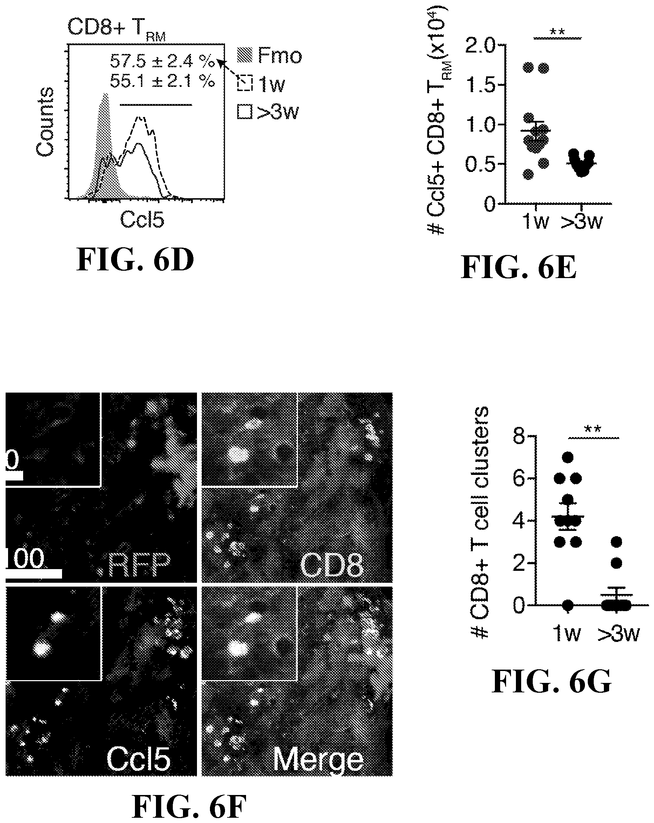

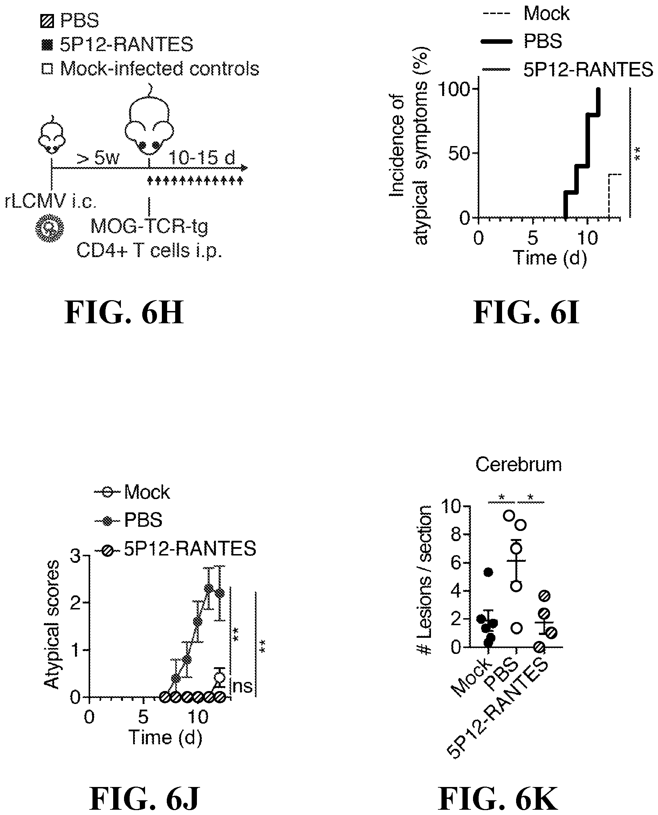

Figure 6 shows that clustering of CCL5+ T_{RM} at sites of previous infection leads to high local CCL5 expression that confers enhanced vulnerability to autoimmune attack. (a) Schematic representation of tissue sampling around RFP+ marker cells for transcriptome analysis. (b) Gene expression analysis of 256 inflammatory genes represented as heatmap. (c) Volcano plot of gene expression data presented in (b). Mice infected at 1w of age are compared to the pool of both control groups. (d) Ex vivo analysis of CCL5-expression by CD8+ brain T_{RM} >5w after rLCMV i.c. infection. (e) Numbers of CCL5+ brain T_{RM} persisting at 5 weeks after rLCMV i.c. infection at 1w or >3w of age. (f) Representative image of clusters of CCL5+ CD8+ T cells in correlation with RFP+ marker cells. (g) Quantification of CD8+ T cell clusters on tissue sections of three different brain levels. (h) Experimental setup for (i-k): Mice were infected i.c. with rLCMV at 1w of age and in vitro-primed 2D2 T cells were transferred >5w later. Mice were treated i.p. with 10 µg 5P12-RANTES or PBS daily after transfer of 2D2 T cells. (i) Incidence and (j) scores of atypical EAE in 5P12-RANTES-treated mice in comparison to PBS-treated littermates or mock-infected controls. (k) Histological analysis of cerebral inflammation two weeks after 2D2 T cell transfer of mice in i, j. e, g, k: Symbols represent individual mice. j: mean ± s.e.m. is shown (n=4-6 mice per group). Data is pooled (e, g) or representative (i-k) of two independent experiments. e, g: Student's t-test, i: Log-rank (Mantel-Cox) test, j: rm-two-way ANOVA, k: One-way ANOVA.

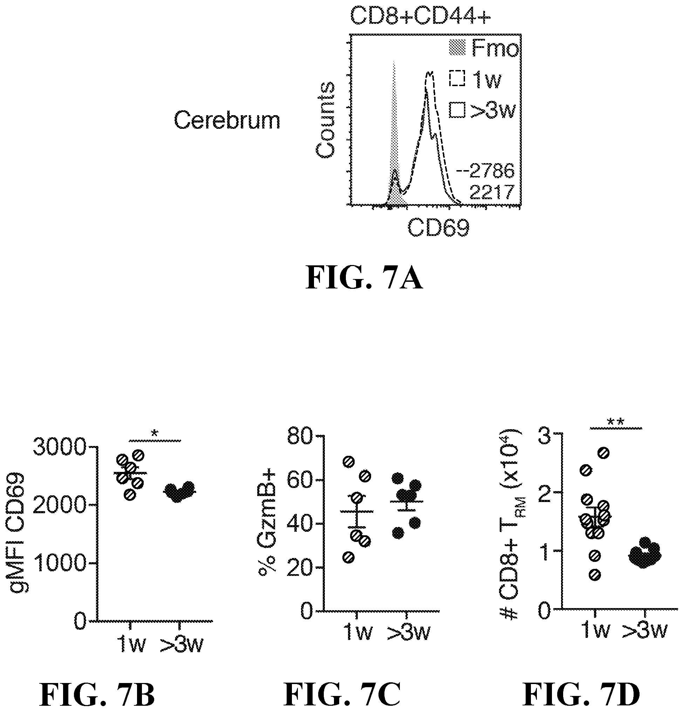

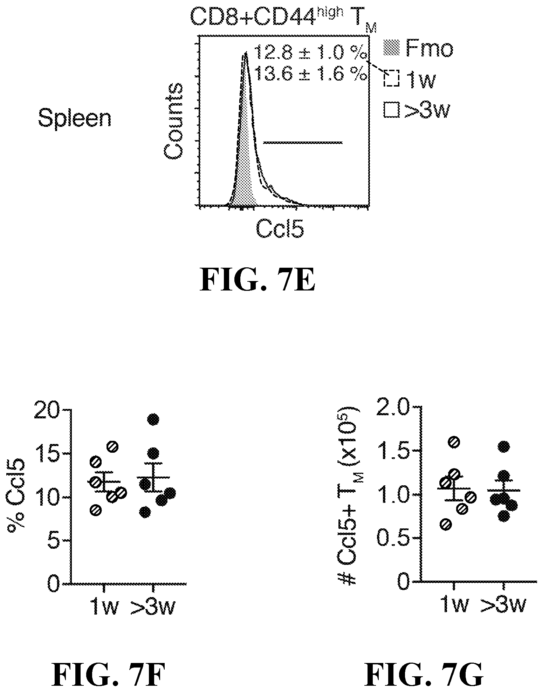

Figure 7 shows characterization of memory T cell subsets in mice infected at 1w or >3w of age. (a-d) Ex vivo flow cytometric characterization and analysis of CD8+CD44+ brain T cells 5w after rLCMV i.c. infection at 1w or >3w of age. (a) Representative histogram of CD69 surface expression in comparison to control staining with normalized gMFI indicated. (b) Quantification of CD69 surface expression (Normalized gMFI). (c) GzmB expression in CD8+CD44+CD69+ brain T_{RM}. (d) Numbers of CD8+CD44+CD69+ brain T_{RM}. (e-g) Ex vivo flow cytometric characterization and analysis of splenic CD8+CD44^{high} memory T cells 5w after rLCMV i.c. infection at 1w or >3w of age. (e) Intracellular staining for CCL5 in comparison to control staining (Fmo). (f) Frequency and (g) numbers of CCL5+ memory T cells, a, b, c, e, f, g: Symbols represent individual mice and mean ± s.e.m. are shown. One representative of two independent experiments. d: Pool of two independent experiments (see Figure 6e). b: Student's t-test.

### DETAILED DESCRIPTION

The present inventors have created an EAE model which assesses cerebral inflammation and closely resembles the pathology of human MS. In particular, the present inventors found that early-in-life viral infection can leave a chronic inflammatory signature that persists long-term after virus clearance in the brain of young mice and facilitates the development of cerebral inflammation during adulthood. This finding may provide a cellular and molecular explanation for the epidemiological studies associating viral infections during childhood with the risk to develop neuroinflammatory disorders later in life.

Using this EAE model, the present inventors have surprisingly discovered that a CCR5 inhibitor, 5P12-RANTES (SEQ ID NO: 70), is capable of inhibiting cerebral inflammation. Moreover, during preclinical evaluation, 5P12-RANTES has been administered to cynomolgus macaques and rats as single dose intravenous infusion at a dose of 20 mg/kg without any adverse events. Accordingly, the claimed CCR5 inhibitors for use in the methods of the present invention as described herein relate to inhibition of cerebral inflammation by administering the claimed CCR5 inhibitor to a subject. The claimed CCR5 inhibitor is provided for use in methods of treating or preventing neuroinflammatory disorders in which cerebral inflammation is involved.

Without being limited by theory, it is believed that CCL5 produced by brain resident memory T cells (bT_{RM}) at sites of resolved brain viral infection early in life promotes precipitation of cerebral inflammation in adulthood, and that blockade of CCL5-CCR5 mediated signaling can inhibit cerebral inflammation and/or abrogate the formation of brain lesions.

As used herein, neuro inflammation is defined as the response of central nervous system (CNS) resident immune cells or infiltrating immune cells to altered homeostasis, whether imposed from inside or outside the CNS, and characterizes all neurological diseases, including developmental, traumatic, ischemic, inflammatory, metabolic, infectious, toxic, neoplastic, and neurodegenerative diseases. Neuroinflammation comprises cerebral inflammation, spinal cord inflammation, or both.

As used herein, inhibition of cerebral inflammation means reducing at least one of: the number of brain lesions of a subject having cerebral inflammation; the incidence of atypical EAE symptoms, such as ataxia or proprioception deficits, in a subject having cerebral inflammation; and the score of atypical EAE in a subject having cerebral inflammation.

In some embodiments, a method of the present invention may reduce the number of brain lesions of a subject having cerebral inflammation by at least about 10%, at least about 20%, at least about 30%, at least about 40%, at least about 50%, at least about 60%, at least about 70%, at least about 80%, at least about 90%, or at least about 99%, compared to a control subject having cerebral inflammation but receiving no treatment.

In some embodiments, a method of the present invention may reduce the incidence of atypical EAE symptoms in a subject having cerebral inflammation by at least about 10%, at least about 20%, at least about 30%, at least about 40%, at least about 50%, at least about 60%, at least about 70%, at least about 80%, at least about 90%, or at least about 99%, compared to a control subject having cerebral inflammation but receiving no treatment.

In some embodiments, a method of the present invention may reduce the score of atypical EAE in a subject having cerebral inflammation by at least about 10%, at least about 20%, at least about 30%, at least about 40%, at least about 50%, at least about 60%, at least about 70%, at least about 80%, at least about 90%, or at least about 99%, compared to a control subject having cerebral inflammation but receiving no treatment.

### CCR5 Inhibitors

As used herein, the term "CCR5 inhibitor" means a molecule or compound that inhibits one or more biological and/or pathological activities induced by CCR5. CCR5 inhibitors may prevent the binding of ligands or pathogens by achieving partial or complete occupation of the site or sites on CCR5 that they require for interaction (orthosteric inhibitors). Alternatively, CCR5 inhibitors may prevent the binding of ligands or pathogens by engaging other sites on CCR5 and inducing CCR5 to adopt a conformation or conformations that cannot be recognized by the ligands or pathogens (allosteric inhibitors). CCR5 inhibitors may inhibit the entire repertoire or only a subset of CCR5 intracellular signaling pathways. CCR5 inhibitors may also act by eliciting long-term intracellular sequestration of CCR5 so that it cannot be accessed by extracellular ligands or pathogens. CCR5 inhibitors may also act by specifically inhibiting or removing expression of the CCR5 gene. CCR5 inhibitors may also act by binding to one or more ligands or pathogens so that they are unable to bind to CCR5.

The CCR5 inhibitors for use according to the claimed invention in the medical method as described herein are N-terminally modified RANTES variants disclosed in US Patent No. 8,686,111, namely polypeptides comprising an N-terminal portion and a C-terminal portion, wherein the N-terminal portion comprises the signature sequence QGP[P or L] and the amino acid sequence of the C-terminal portion is at least 70% identical to SEQ ID NO: 1.

In some embodiments, pharmaceutical compositions are provided which may comprise more than one CCR5 inhibitor as described herein, e.g. two or more CCR5 inhibitors.

CCR5 inhibitors that may be used in the methods of the present invention include, but are not limited to, antibodies that bind to CCL5 (such as those disclosed in Glass, W. G. et al. J. Immunol. 172, 4018-4025 (2004); Kennedy, K.J. et al. J. Neuroimmunol. 92, 98-108 (1998); Youssef, S. et al. J. Immunol. 161, 3870-3879 (1998); and WO2017058896); TAK-779 (dimethyl-[[4-[[3-(4-methylphenyl)-8,9-dihydro-7H-benzo[7]annulene-6-carbonyl]amino]phenyl]methyl]-(oxan-4-yl)azanium chloride); mimic peptides that specifically bind with the first and second extracellular loops (ECL1, ECL2) of CCR5 disclosed in Zheng, H.M. et al. Inflamm. Res. 60, 759-767 (2011); N-terminally modified/truncated RANTES variants (such as Met-RANTES, AOP-RANTES, NNY-RANTES and PSC-RANTES, the sequences of which are depicted in Table 1 below, as well as those disclosed in US Patent Nos. 6,942,852; 7,326,411; 7,915,221; and 8,686,111 and PCT Patent Application Publication No. WO1998006751); RANTES variants containing mutations in the N-loop and/or 40's loop disclosed in US Patent Nos. 7,164,000; 7,335,350; and 7,402,303; RANTES-derived peptides disclosed in US Patent Application Publication No. 20060165650; the CCR5 inhibitors disclosed in US Patent Application Publication No. 20070270429; maraviroc (4,4-difluoro-*N*-{(1*S*)-3-[*exo*-3-(3-isopropyl-5-methyl-4*H*-1,2,4-triazol-4-yl)-8-azabicyclo[3.2.1]oct-8-yl]-1-phenylpropyl}cyclohexanecarboxamide); vicriviroc 4,6-dimethylpyrimidin-5-yl)-[4-[(3*S*)-4-[(1*R*)-2-methoxy-1-[4-(trifluoromethyl)phenyl]ethyl]-3-methylpiperazin-1-yl]-4-methylpiperidin-1-yl]methanone); cenicriviroc ((5*E*)-8-[4-(2-butoxyethoxy)phenyl]-1-(2-methylpropyl)-*N*-[4-[(*S*)-(3-propylimidazol-4-yl)methylsulfinyl]phenyl]-3,4-dihydro-2*H*-1-benzazocine-5-carboxamide); aplaviroc (4-[4-[[(3*R*)-1-butyl-3-[(*R*)-cyclohexyl(hydroxy)methyl]-2,5-dioxo-1,4,9-triazaspiro[5.5]undecan-9-yl]methyl]phenoxy]benzoic acid); and INCB9471 ((4,6-dimethyl-5-pyrimidinyl)(4-{(3*S*)-4-[(1*R*,2*R*)-2-ethoxy-5-(trifluoromethyl)-2,3-dihydro-1*H*-inden-1-yl]-3-methyl-1-piperazinyl} -4-methyl-1-piperidinyl)methanone).

In some embodiments, CCR5 inhibitors that may be used in the methods of the present invention are highly potent inhibitors of HIV entry into cells, i.e. have high anti-HIV potency. In the context of the present invention, the expressions "high anti-HIV potency", "high potency" or "highly potent" are used with regard to CCR5 inhibitors having an IC50 value, as measured by the cell fusion or HIV replication assay as described in the Materials and Methods section of US Patent No. 8,686,111, of 1000 pM (1 nM) or lower, for example, less than 900, 800, 700, 600, 500, 400, 300, 200, 150, 140, 130, 120, 110, 100, 90, 80, 70, 60, 50, 40, 30, or 20 pM.

In some embodiments, CCR5 inhibitors that may be used in the methods of the present invention have low signaling activity, i.e., their administration and/or binding to CCR5 causes only a low degree of pro-inflammatory signaling in target cells. In the context of the present invention, CCR5 inhibitors with "low signaling activity" lead to a signaling response of 30% or less of the maximum response (Emax) elicited by PSC-RANTES, when tested at a concentration of 300 nM in the Calcium Flux signaling assay as described in the Materials and Methods section of US Patent No. 8,686,111. For example, CCR5 inhibitors used in the methods of the present invention may have signaling activities, as measured in the Calcium Flux signaling assay, of less than 30%, 26%, 22%, 20%, 18%, 16%, 14%, 12%, 10%, 8%, 6%, 4%, 2% or 1% of the maximum response (Emax) elicited by PSC-RANTES.

In some embodiments, CCR5 inhibitors that may be used in the methods of the present invention are selective for CCR5 over the receptors CCR1 and/or CCR3. In the context of the present invention, a CCR5 inhibitor that binds to CCR1 and/or CCR3 as well as CCR5 is still considered to have selectivity for CCR5 over CCR1 and/or CCR3 if it does not substantially activate CCR1 and/or CCR3. In some embodiments, CCR5 inhibitors that may be used in the methods of the present invention neither substantially bind nor substantially activate CCR1 and CCR3. In the context of the present invention, a CCR5 inhibitor is considered not to substantially bind CCR1 and/or CCR3 if the IC50 value of the CCR5 inhibitor with respect to CCR1 and/or CCR3 binding is greater than 50 nM, for example, greater than 60, 70, 80, 90, 100, 110, 120, 130, 140, 150, 200, 300, 400, 500, 600, 700, 800, 900 or 1000 nM, as measured using the CCR1 Discrimination Binding Assay and/or the CCR3 Discrimination Binding Assay as described in the Materials and Methods section of US Patent No. 8,686,111. Selectivity of CCR5 activation over CCR1 and/or CCR3 activation may be evaluated by means of the Calcium Flux Assay as described in the Materials and Methods section of US Patent No. 8,686,111. In the context of the present invention, a CCR5 inhibitor is considered not to substantially activate CCR1 and/or CCR3 if its signaling activity is less than 30%, more preferably less than 26%, 22%, 20%, 18%, 16%, 14%, 12%, 10%, 8%, 6%, 4%, 2% or 1% of the Emax elicited on that receptor by native RANTES/CCL5, as measured by the Calcium Flux Assay as described in the Materials and Methods section of US Patent No. 8,686,111.

In some embodiments, CCR5 inhibitors that may be used in the methods of the present invention have high receptor sequestration activity, i.e. administration and/or binding to CCR5 causes a high degree of receptor sequestration. Said sequestration may be receptor internalisation, down-regulation or down-modulation. In the context of the present invention, CCR5 inhibitors with "high receptor sequestration activity" lead to the sequestration of at least 50% of the control level of surface CCR5 molecules, when tested in the CCR5 Surface Downmodulation/receptor sequestration assay as described in the Materials and Methods section of US Patent No. 8,686,111. For example, CCR5 inhibitors may have receptor sequestration activities of more than 50%, 55%, 60%, 65%, 70%, 80%, 85%, 90%, or 95% of the control level of surface CCR5.

In some embodiments, CCR5 inhibitors that may be used in the methods of the present invention combine high anti-HIV potency with low signaling activity, or combine high anti-HIV potency with high receptor sequestration activity. In some embodiments, CCR5 inhibitors that may be used in the methods of the present invention combine high anti-HIV potency with both low signaling and high receptor sequestration activity. In some embodiments, CCR5 inhibitors that may be used in the methods of the present invention combine at least one property selected from the group consisting of high anti-HIV potency, high receptor sequestration activity and low signaling activity with selectivity for CCR5.

In some embodiments, CCR5 inhibitors that may be used in the methods of the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with low levels of signaling activity (e.g., no more than 15%, no more than 10%, or no more than 5%, as measured by the Calcium Flux Assay as described in the Materials and Methods section of US Patent No. 8,686,111) and low levels of receptor sequestration activity (e.g., no more than 20%, or no more than 10%, as measured by the CCR5 Surface Downmodulation Assay as described in the Materials and Methods section of US Patent No. 8,686,111).

In some embodiments, CCR5 inhibitors that may be used in the methods of the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with high levels of signaling activity (e.g., at least 60%, or at least 85% as measured by the Calcium Flux Assay as described in the Materials and Methods section of US Patent No. 8,686,111) and high levels of receptor sequestration activity (e.g., at least 60%, or at least 70%, as measured by the CCR5 Surface Downmodulation Assay as described in the Materials and Methods section of US Patent No. 8,686,111).

In some embodiments, CCR5 inhibitors that may be used in the methods of the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with low levels of signaling activity (e.g., no more than 30%, or no more than 20%, no more than 15%, or no more than 10%, as measured by the Calcium Flux Assay as described in the Materials and Methods section of US Patent No. 8,686,111) and intermediate levels of receptor sequestration activity (e.g., between 20% and 50%, or between 30% and 50%, as measured by the CCR5 Surface Downmodulation Assay as described in the Materials and Methods section of US Patent No. 8,686,111).

In some embodiments, CCR5 inhibitors that may be used in the methods of the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with low levels of signaling activity (e.g., no more than 30%, or no more than 20%, no more than 15%, or no more than 10%, as measured by the Calcium Flux Assay as described in the Materials and Methods section of US Patent No. 8,686,111) and high levels of receptor sequestration activity (e.g., at least 50%, as measured by the CCR5 Surface Downmodulation Assay as described in the Materials and Methods section of US Patent No. 8,686,111).

In some embodiments, CCR5 inhibitors that may be used in the methods of the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with intermediate levels of signaling activity (e.g., between 30% and 50%, or between 30% and 45%, as measured by the Calcium Flux Assay as described in the Materials and Methods section of US Patent No. 8,686,111) and high levels of receptor sequestration activity (e.g., at least 50%, as measured by the CCR5 Surface Downmodulation Assay as described in the Materials and Methods section of US Patent No. 8,686,111).

In some embodiments, CCR5 inhibitors that may be used in the methods of the present invention are characterised by a combination of intermediate levels of anti-HIV potency (IC50 levels, as measured by the Cell Fusion Assay as described in the Materials and Methods section of US Patent No. 8,686,111, of e.g., between 0.02 and 1 nM, between 0.02 and 0.15 nM, or between 0.5 and 1 nM) with intermediate levels of signaling activity (e.g., between 30% and 50%, or between 30% and 45% as measured by the Calcium Flux Assay as described in the Materials and Methods section of US Patent No. 8,686,111) and intermediate levels of receptor sequestration activity (e.g., between 20% and 50%, or between 30% and 50%, as measured by the CCR5 Surface Downmodulation Assay as described in the Materials and Methods section of US Patent No. 8,686,111).

In some embodiments, CCR5 inhibitors that may be used in the methods of the present invention include RANTES variants pVU5, pVU14, and pVU43 disclosed in US Patent Nos. 7,164,000, the sequences of which are depicted in Table 1 below; RANTES (9-68) disclosed in PCT Patent Application Publication No. WO1998006751, the sequence of which is depicted in Table 1 below; RANTES-derived peptides disclosed in US Patent Application Publication No. 20060165650; J113863 (1,4-*cis*-1-(1-cycloocten-1-ylmethyl)-4-[[(2,7-dichloro-9*H*-xanthen-9-yl)carbonyl]amino]-1-ethylpiperidinium iodide), USB35625 (1,4-*trans*-1-(1-cycloocten-1-ylmethyl)-4-[[(2,7-dichloro-9*H-*xanthen-9-yl)carbonyl]amino]-1-ethylpiperidinium iodide), neoabietic acid (8(14), 13(15)-abietadien-18-oic-acid), (4-[4-({(3*R*)-1-butyl-3-[(*R*)-cyclohexyl(hydroxy)methyl]-2,5-dioxo-1,4,9-triazaspiro[5.5]undec-9-yl}methyl)benzyl]-3-methoxybenzoic acid), and 4-[4-({(3*R*)-1-butyl-3-[(*R*)-cyclohel(hydroxy)methyl]-2,5-dioxo-1,4,9-triazaspiro[5.5]undec-9-yl}methyl)phenoxy]-3-ethoxybenzoic acid hydrochloride disclosed in US Patent Application Publication No. 20070270429; Met-RANTES; P1 disclosed in US Patent No. 7,915,221, the sequence of which is depicted in Table 1 below; maraviroc; vicriviroc; cenicriviroc; aplaviroc; INCB9471; TAK-779; and N-terminally modified RANTES variants disclosed in US Patent No. 8,686,111.

**TABLE 1**

| | |
|---|---|
| NAME | SEQUENCE |
| Met-RANTES | MSPYSSDTTPCCFAYIARPLPRAHIKEYFYTSGKCSNPAVVFVTRKNR QVCANPEKKWVREYINSLEMS (SEQ ID NO: 73) |
| | |
| AOP-RANTES | R¹PYSSDTTPCCFAYIARPLPRAHIKEYFYTSGKCSNPAVVFVTRKNRQ VCANPEKKWVREYINSLEMS, where R¹ = CH₃–(CH₂)₄–O–N=CH–C(O)– (SEQ ID NO: 74) |
| | |
| NNY-RANTES | R¹PYSSDTTPCCFAYIARPLPRAHIKEYFYTSGKCSNPAVVFVTRKNRQ VCANPEKKWVREYINSLEMS, where R¹ = CH₃–(CH₂)₇–C(O)– (SEQ ID NO: 75) |
| | |
| PSC-RANTES | R¹SSDTTPCCFAYIARPLPRAHIKEYFYTSGKCSNPAVVFVTRKNRQV CANPEKKWVREYINSLEMS, where R¹ = Nα-(n-nonanoyl)-des-Ser¹-[L- thioprolyl², L-cyclohexylglycyl³] (SEQ ID NO: 76) |
| | |
| pVU5 | CPYSSDTTPCCFAYIARPLPRAHIKEYFYTSGKCSNPAVVFVTRKNRQ VCANPEKKWVREYINSLEMS (SEQ ID NO: 77) |
| | |
| pVU14 | SPASSDTTPCCFAYIARPLPRAHIKEYFYTSGKCSNPAVVFVTRKNRQ |
| | VCANPEKKWVREYINSLEMS (SEQ ID NO: 78) |
| | |
| pVU43 | SPYSSDTTPCCFAYIARPLPRAHIKEYFYTSGKCSNPAVVFVTRENRQ VCANPEKKWVREYINSLEMS (SEQ ID NO: 79) |
| | |
| RANTES (9-68) | CCFAYIARPLPRAHIKEYFYTSGKCSNPAVVFVTRKNRQVCANPEKK WVREYINSLEMS (SEQ ID NO: 80) |
| | |
| P1 | LSPVSSQSSACCFAYIARPLPRAHIKEYFYTSGKCSNPAVVFVTRKNR QVCANPEKKWVREYINSLEMS (SEQ ID NO: 81) |

In some embodiments, CCR5 inhibitors that may be used in the methods of the present invention include Met-RANTES; P1; maraviroc; vicriviroc; cenicriviroc; aplaviroc; INCB9471; TAK-779; and N-terminally modified RANTES variants disclosed in US Patent No. 8,686,111.

CCR5 inhibitors for use according to the claimed invention that are always used in the methods of the present invention are disclosed in US Patent No. 8,686,111, and are polypeptides that comprise an N-terminal portion and a C-terminal portion, wherein the N-terminal portion comprises the signature sequence QGP[P or L], i.e., the fourth the N-terminal portion comprises the signature sequence QGP[P or L], i.e., the fourth position of the signature sequence may be either P or L, and the amino acid sequence of the C-terminal portion is at least 70% identical to SEQ ID NO: 1. The polypeptides disclosed in US Patent No. 8,686,111 may combine high anti-HIV potency with a capacity to elicit only a low degree of pro-inflammatory signaling and/or a capacity to lead to the internalisation of CCR5 into the cell (receptor sequestration, down-regulation or down-modulation).

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or G or S or M][M or D or S or Q or G]; QGP[P or L][L or G][M or D or S]; QGP[P or L][L or G or S or M][M or D or S or Q or G]XX[Q or G or L or A or T or S]X, wherein X denotes any natural or modified amino acid; QGP[P or L] [L or G][M or D or S]XX[Q or L]X, wherein X denotes any natural or modified amino acid; QGP[P or L] LM; QGPPG[D or S]; QGPPLM; or QGPPGD.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or M][M or Q][A or W or G or Q or N]X[Q or G or L][S or V or T or G], wherein X denotes any natural or modified amino acid; QGP[P or L][L or M] [M or Q][A or W or G or Q or N] [L or T or M or S or G or Q or R or Y][Q or G or L][S or V or T or G]; QGP[P or L]LM[A or W][L or T or M][Q or G][S or V or T or G]; or QGPPLM[A or W] [L or T or M][Q or G] [S or V or T or G].

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or G or S][D or S or G or Q] XX[L or A or T or Q][W or A or V], wherein X denotes any natural or modified amino acid; QGP[P or L][L or G or S][D or S or G or Q][T or I or S or W or Q] [V or L or A or S or G][L or A or T or Q][W or A or V]; QGPPG[D or S][T or I]VL[W or A]; or QGPPGD[T or I]VL[W or A].

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGPP[G or L][M or Q]XX[Q or S][S or V], wherein X denotes any natural or modified amino acid; QGPP[G or L][M or Q][S or G or W or A or T][L or For T or S or G or Y][Q or S][S or V]; or QGPPLM[S or G][L or For T]Q[S or V].

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMALQS, QGPPLMWMQV, QGPPLMWLQV, QGPPLMWTQS, QGPPLMWLQT, QGPPLMWTQV, QGPPLMWMQS, QGPPLMATQS, QGPPLMWLQS, QGPPLMALQV, QGPPLMWLGG, QGPPLMWRGS, QGPLLMWLQV, QGPPLMQTTP, QGPPLSWLQV, QGPPLSWLQS, QGPPGQWSQV, QGPPMMAGLS, QGPPLSWQQS, QGPPGMWSQS, QGPPLQWRQS, QGPPLMGTQS, QGPPLMQLQV, QGPPLSWSQV, QGPPMSWSQS, QGPPLMNLQV, QGPPMSAYQV and QGPPMQGGLS.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMALQS, QGPPLMWMQV, QGPPLMWLQV, QGPPLMWTQS, QGPPLMWLQT, QGPPLMWTQV, QGPPLMWMQS, QGPPLMATQS, QGPPLMWLQS, QGPPLMALQV, QGPPLMWLGG, QGPPLMWRGS, QGPLLMWLQV, and QGPPLMQTTP.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPGDTVLW, QGPPGDIVLA, QGPPGSYDYS, QGPPGDGGSV, QGPLSGQSTP, QGPPGDWLQV, QGPPLMSLAV, QGPPLMSLTV, QGPLSGWAQV, QGPLSQSSQV, QGPLSSQSQV and QGPLGQQGQV.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPGDTVLW, QGPPGDIVLA, QGPPGSYDYS, QGPPGDGGSV, QGPLSGQSTP, and QGPPGDWLQV.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSFQS, QGPPLMSTQS, QGPPLMSLQV, QGPPLMGLQV, QGPLSGWLQV, QGPPLQWFQV, QGPPLQWTQV, QGPPLMALSV, QGPPLMWSQV, QGPPGQWGQV, QGPPGSWSQV, QGPPLMSSQS, QGPPLMGLSV, QGPPLMTLQV and QGPPGQWYQS.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSFQS, QGPPLMSTQS, QGPPLMSLQV, QGPPLMGLQV, and QGPLSGWLQV.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSVLA, QGPPGSWSSV, QGPPLGSMGP, QGPPLQWMQA, QGPPLQWMQV, QGPPLMSTQV, QGPPLMSLSV, QGPPLMSLQS, QGPPLMSLQA, QGPPLMSVQS, QGPPLMSAQS, QGPPLMSGQS and QGPPLMSGQV.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSVLA, QGPPGSWSSV, and QGPPLGSMGP.

In some embodiments, CCR5 inhibitors that may be used in the methods of the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the signature sequences set out in Table 2:

**TABLE 2**

| SEQ ID NO | Signature Sequence |
|---|---|
| SEQ ID NO: 2 | QGPPLMALQS |
| SEQ ID NO: 3 | QGPPLMWMQV |
| SEQ ID NO: 4 | QGPPLMWLQV |
| SEQ ID NO: 5 | QGPPLMWTQS |
| SEQ ID NO: 6 | QGPPLMWLQT |
| SEQ ID NO: 7 | QGPPLMWTQV |
| SEQ ID NO: 8 | QGPPLMWMQS |
| SEQ ID NO: 9 | QGPPLMATQS |
| SEQ ID NO: 10 | QGPPLMWLQS |
| SEQ ID NO: 11 | QGPPLMALQV |
| SEQ ID NO: 12 | QGPPLMWLGG |
| SEQ ID NO: 13 | QGPPLMWRGS |
| SEQ ID NO: 14 | QGPLLMWLQV |
| SEQ ID NO: 15 | QGPPLMQTTP |
| SEQ ID NO: 16 | QGPPGDTVLW |
| SEQ ID NO: 17 | QGPPGDIVLA |
| SEQ ID NO: 18 | QGPPGSYDYS |
| SEQ ID NO: 19 | QGPPGDGGSV |
| SEQ ID NO: 20 | QGPLSGQSTP |
| SEQ ID NO: 21 | QGPPGDWLQV |
| SEQ ID NO: 22 | QGPPLMSFQS |
| SEQ ID NO: 23 | QGPPLMSTQS |
| SEQ ID NO: 24 | QGPPLMSLQV |
| SEQ ID NO: 25 | QGPPLMGLQV |
| SEQ ID NO: 26 | QGPLSGWLQV |
| SEQ ID NO: 27 | QGPPLMSVLA |
| SEQ ID NO: 28 | QGPPGSWSSV |
| SEQ ID NO: 29 | QGPPLGSMGP |
| SEQ ID NO: 30 | QGPPLSWLQV |
| SEQ ID NO: 31 | QGPPLSWLQS |
| SEQ ID NO: 32 | QGPPGQWSQV |
| SEQ ID NO: 33 | QGPPMMAGLS |
| SEQ ID NO: 34 | QGPPLSWQQS |
| SEQ ID NO: 35 | QGPPGMWSQS |
| SEQ ID NO: 36 | QGPPLQWRQS |
| SEQ ID NO: 37 | QGPPLMGTQS |
| SEQ ID NO: 38 | QGPPLMQLQV |
| SEQ ID NO: 39 | QGPPLSWSQV |
| SEQ ID NO: 40 | QGPPMSWSQS |
| SEQ ID NO: 41 | QGPPLMNLQV |
| SEQ ID NO: 42 | QGPPMSAYQV |
| SEQ ID NO: 43 | QGPPMQGGLS |
| SEQ ID NO: 44 | QGPPLMSLAV |
| SEQ ID NO: 45 | QGPPLMSLTV |
| SEQ ID NO: 46 | QGPLSGWAQV |
| SEQ ID NO: 47 | QGPLSQSSQV |
| SEQ ID NO: 48 | QGPLSSQSQV |
| SEQ ID NO: 49 | QGPLGQQGQV |
| SEQ ID NO: 50 | QGPPLQWFQV |
| SEQ ID NO: 51 | QGPPLQWTQV |
| SEQ ID NO: 52 | QGPPLMALSV |
| SEQ ID NO: 53 | QGPPLMWSQV |
| SEQ ID NO: 54 | QGPPGQWGQV |
| SEQ ID NO: 55 | QGPPGSWSQV |
| SEQ ID NO: 56 | QGPPLMSSQS |
| SEQ ID NO: 57 | QGPPLMGLSV |
| SEQ ID NO: 58 | QGPPLMTLQV |
| SEQ ID NO: 59 | QGPPGQWYQS |
| SEQ ID NO: 60 | QGPPLQWMQA |
| SEQ ID NO: 61 | QGPPLQWMQV |
| SEQ ID NO: 62 | QGPPLMSTQV |
| SEQ ID NO: 63 | QGPPLMSLSV |
| SEQ ID NO: 64 | QGPPLMSLQS |
| SEQ ID NO: 65 | QGPPLMSLQA |
| SEQ ID NO: 66 | QGPPLMSVQS |
| SEQ ID NO: 67 | QGPPLMSAQS |
| SEQ ID NO: 68 | QGPPLMSGQS |
| SEQ ID NO: 69 | QGPPLMSGQV |

In one embodiment, the CCR5 inhibitor used in the methods of the present invention comprises an N-terminal portion and a C-terminal portion, wherein the N-terminal portion comprises the signature sequence QGPPLMATQS and the amino acid sequence of the C-terminal portion is at least 70% identical to SEQ ID NO: 1.

In one embodiment, the CCR5 inhibitor used in the methods of the present invention comprises an N-terminal portion and a C-terminal portion, wherein the N-terminal portion comprises the signature sequence QGPPGDIVLA and the amino acid sequence of the C-terminal portion is at least 70% identical to SEQ ID NO: 1.

In one embodiment, the CCR5 inhibitor used in the methods of the present invention comprises an N-terminal portion and a C-terminal portion, wherein the N-terminal portion comprises the signature sequence QGPPLMSLQV and the amino acid sequence of the C-terminal portion is at least 70% identical to SEQ ID NO: 1.

In one embodiment, the CCR5 inhibitor used in the methods of the present invention comprises an N-terminal portion and a C-terminal portion, wherein the N-terminal portion consists of the signature sequence QGPPLMATQS and the amino acid sequence of the C-terminal portion is SEQ ID NO: 1.

In one embodiment, the CCR5 inhibitor used in the methods of the present invention comprises an N-terminal portion and a C-terminal portion, wherein the N-terminal portion consists of the signature sequence QGPPGDIVLA and the amino acid sequence of the C-terminal portion is SEQ ID NO: 1.

In one embodiment, the CCR5 inhibitor used in the methods of the present invention comprises an N-terminal portion and a C-terminal portion, wherein the N-terminal portion consists of the signature sequence QGPPLMSLQV and the amino acid sequence of the C-terminal portion is SEQ ID NO: 1.

In one embodiment, the CCR5 inhibitor used in the methods of the present invention comprises the amino acid sequence of SEQ ID NO: 70.

In one embodiment, the CCR5 inhibitor used in the methods of the present invention comprises the amino acid sequence of SEQ ID NO: 71.

In one embodiment, the CCR5 inhibitor used in the methods of the present invention comprises the amino acid sequence of SEQ ID NO: 72.

The N-terminal portion of the CCR5 inhibitors described in paragraphs [0059] to [0075] may consist of no more than 15, 14, 13, 12, 11, or 10 amino acids. In one embodiment, said N-terminal portion consists of 10 amino acids.

In the CCR5 inhibitors described in paragraphs [0059] to [0075], the N-terminus of the C-terminal portion may adjoin directly to the C-terminus of the N-terminal portion, i.e. the N-terminal portion and the C-terminal portion are directly adjoined.

The C-terminal portion of the CCR5 inhibitors described in paragraphs [0059] to [0075] may have more than 70%, 75%, 80%, 85%, 90%, 95%, 98%, 99%, 99.9% or 100% sequence identity to SEQ ID NO: 1.

The term "sequence identity," as used herein, has the standard meaning in the art. As is known in the art, a number of different programs can be used to identify whether a polynucleotide or polypeptide has sequence identity or similarity to a known sequence. Sequence identity or similarity may be determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387 (1984), preferably using the default settings, or by inspection.

The signature sequence of the CCR5 inhibitors described in paragraphs [0059] to [0075] may be located near the N-terminus of the polypeptide. For example, the signature sequence may be located such that the beginning of the signature sequence lies within 15, 12, 10, 8, 6, 5, 4, 3, 2, or 1 residue of the N-terminus of the polypeptide. Herein, the expression "the beginning of the signature sequence" refers to the N-terminus of the signature sequence. The signature sequence may also be located at the extreme N-terminus of the polypeptide, i.e., the N-termini of the polypeptide as a whole and the signature sequence may coincide.

In some embodiments, nucleic acids encoding the polypeptides provided herein may be used in the methods of the present invention as described herein. The skilled person would know how to design or identify nucleic acids encoding the polypeptides, according to the genetic code. Such a nucleic acid may comprise one or more segments encoding one or more polypeptides provided herein. Such a nucleic acid may be RNA or DNA. Such a nucleic acid may be a vector, i.e. nucleic acids encoding the polypeptides provided herein may be incorporated within a vector.

In some embodiments, nucleic acids encoding the polypeptides provided herein may be incorporated into a virus. Thus, a virus that contains, within its genome, one or more segments encoding one or more polypeptides provided herein, may be used in the methods of the present invention as described herein.

As used herein, the terms "protein", "peptide" or "polypeptide" are used interchangeably and refer to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labelling component. Also included within the definition are, for example, polypeptides containing one or more analogues of an amino acid (including, for example, unnatural amino acids, etc.), as well as other modifications known in the art. Polypeptides can occur as single chains or associated chains. Polypeptides of the invention can be naturally or non-naturally glycosylated (i.e. the polypeptide has a glycosylation pattern that differs from the glycosylation pattern found in the corresponding naturally occurring polypeptide).

### Pharmaceutical Compositions and Dosage Forms

The present invention provides compositions, for example, pharmaceutical compositions, comprising CCR5 inhibitors as described herein. As used herein, the terms "pharmaceutical composition" and "composition" may be used interchangeably, as the context requires. A pharmaceutical composition as disclosed herein may be administered to a subject in a therapeutically effective amount. As used herein, a "therapeutically effective amount" means an amount of the composition or therapeutic agent effective to provide a therapeutic, prophylactic or diagnostic benefit to a subject. In some embodiments, a therapeutically effective amount of the composition is an amount capable of inducing a clinical response in a subject in the treatment of a particular disease or disorder. Determination of a therapeutically effective amount of the composition is well within the capability of those skilled in the art, especially in light of the disclosure provided herein. The therapeutically effective amount may vary according to a variety of factors such as the subject's condition, weight, sex and age.

Pharmaceutical compositions provided herein may further comprise a pharmaceutically acceptable carrier, excipient, and/or stabilizer (Remington: The Science and practice of Pharmacy 20th Ed., 2000, Lippincott Williams and Wilkins, Ed. K. E. Hoover). Acceptable carriers, excipients, or stabilizers are nontoxic to recipients at the dosages and concentrations, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

In some embodiments, pharmaceutical compositions provided herein may comprise more than one CCR5 inhibitor as described herein, e.g., two or more CCR5 inhibitors. In some embodiments, pharmaceutical compositions provided herein may comprise a second therapeutic agent, for example, an anti-inflammatory drug or an immunosuppressant. As used herein, the term "therapeutic agent" is any molecule, substance or compound that is capable of providing a therapeutic activity, response or effect in the treatment or prevention of a disease, disorder or condition, including diagnostic and prophylactic agents.

Pharmaceutical compositions provided herein may be prepared in various pharmaceutical dosage forms, such as an instant release, controlled release, sustained release, or target drug-delivery system. Commonly used dosage forms include, for example, solutions and suspensions, (micro-) emulsions, ointments, gels, creams, pastes, foams, suppositories, ovules, implants, patches, liposomes, tablets, dragees, lozenges, soft or hard shell capsules, amorphous or crystalline powders, effervescent powders or tablets, aerosols, and lyophilized formulations. Depending on the route of administration used, special devices may be required for application or administration of a dosage form, such as syringes and needles, inhalers, pumps, injection pens, applicators, special flasks, or other devices for administration, which may also be implanted within a body.

Pharmaceutical dosage forms provided herein may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes.

For the methods of the present invention, CCR5 inhibitors, pharmaceutical compositions or dosage forms provided herein may be administered to a subject by conventional techniques, such as intravenously (as a bolus or by continuous infusion over a period of time), intramuscularly, transmucosally, intraperitoneally, intracerebrally, subcutaneously, intra-articularly, intrasynovially, intrathecally, nasally, orally, topically, or by inhalation. Other suitable administration routes may include intra-lesional or peri-lesional routes.

For intravenous injection, for example, pharmaceutical compositions provided herein may be formulated in aqueous solution, if necessary using physiologically compatible buffers, including, for example, phosphate, histidine, or citrate for adjustment of the formulation pH, and a tonicity agent, such as, for example, sodium chloride or dextrose. For transmucosal or nasal administration, semisolid, liquid formulations, or patches may be preferred, possibly containing penetration enhancers. Such penetrants are generally known in the art. For oral administration, pharmaceutical compositions provided herein may be formulated in liquid or solid dosage forms and optionally as instant or controlled/sustained release formulations. Suitable dosage forms for oral ingestion by a subject include tablets, capsules, pills, dragees, hard and soft shell capsules, liquids, gels, syrups, slurries, suspensions, and emulsions.

Solid oral dosage forms can be obtained using excipients, which may include inert diluents, fillers, disintegrants, binders (dry and wet), dissolution retardants, lubricants, glidants, antiadherants, cationic exchange resins, wetting agents, antioxidants, preservatives, colouring, sweetening and flavouring agents. These excipients can be of synthetic or natural source. Examples of such excipients include cellulose derivatives, citric acid, dicalcium phosphate, gelatine, magnesium carbonate, magnesium/sodium lauryl sulfate, mannitol, polyethylene glycol, polyvinyl pyrrolidone, silicates, silicon dioxide, sodium benzoate, sorbitol, starches, stearic acid or a salt thereof, sugars (i.e. dextrose, sucrose, lactose, etc.), talc, tragacanth mucilage, vegetable oils (hydrogenated), and waxes. Ethanol and water may serve as granulation aides. In certain instances, coating of tablets with, for example, a taste-masking film, a stomach acid resistant film, or a release-retarding film is desirable. Natural and synthetic polymers, in combination with colorants, sugars, and organic solvents or water, are often used to coat tablets, resulting in dragees. When a capsule is preferred over a tablet, the drug powder, suspension, or solution thereof can be delivered in a compatible hard or soft shell capsule.

Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatine capsules in which an active ingredient is mixed with a solid diluent and soft gelatine capsules wherein the active ingredient is mixed with water or an oil such as peanut oil, liquid paraffin or olive oil.

In some embodiments, CCR5 inhibitors provided herein may be administered topically, via the skin or mucous membrane, such as through a skin patch, a semi-solid or a liquid formulation, for example a gel, a (micro-) emulsion, an ointment, a solution, a (nano/micro)-suspension, or a foam. The penetration of an active ingredient into the skin or mucous membrane and underlying tissues of a subject can be regulated, for example, using penetration enhancers; the appropriate choice and combination of lipophilic, hydrophilic, and amphiphilic excipients, including water, organic solvents, waxes, oils, synthetic and natural polymers, surfactants, emulsifiers; by pH adjustment; and use of complexing agents.

In some embodiments, CCR5 inhibitors provided herein may be administered by inhalation, or to the nose, in the form of a solution, suspension, emulsion, or semisolid aerosol from pressurized packs, or a nebuliser, usually with the use of a propellant, e.g., halogenated carbons derived from methane and ethane, carbon dioxide, or any other suitable gas. For topical aerosols, hydrocarbons like butane, isobutene, and pentane are useful. In the case of a pressurized aerosol, the appropriate dosage unit may be determined by providing a valve to deliver a metered amount. Capsules and cartridges of, for example, gelatin, for use in an inhaler or insufflator, may be formulated. These typically contain a powder mix of an active ingredient and a suitable powder base such as lactose or starch.

Compositions formulated for parenteral administration by injection are usually sterile and, can be presented in unit dosage forms, e.g., in ampoules, syringes, injection pens, or in multi-dose containers, the latter usually containing a preservative. Pharmaceutical compositions suitable for parenteral administration may take such forms as suspensions, solutions, or emulsions in oily or aqueous vehicles, and may contain formulatory agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Depot formulations, providing controlled or sustained release of an active agent, may include injectable suspensions of nano/micro particles or nano/micro or non-micronized crystals. Polymers such as poly(lactic acid), poly(glycolic acid), or copolymers thereof, can serve as controlled/sustained release matrices, in addition to others well known in the art. Other depot delivery systems may be presented in form of implants and pumps requiring incision.

Suitable carriers for intravenous injection of CCR5 inhibitors provided herein are well-known in the art and include water-based solutions containing a base, such as, for example, sodium hydroxide, to form an ionized agent, sucrose or sodium chloride as a tonicity agent. A water-based solution may comprise a buffer containing phosphate or histidine. Co-solvents, such as polyethylene glycols, may be added. These water-based systems are effective at dissolving agents and produce low toxicity upon systemic administration. The proportions of the components of a solution system may be varied considerably, without destroying solubility and toxicity characteristics. Furthermore, the identity of the components may be varied. For example, low-toxicity surfactants, such as polysorbates or poloxamers, may be used, as can polyethylene glycol or other co-solvents, biocompatible polymers such as polyvinyl pyrrolidone may be added, and other sugars and polyols may substitute for dextrose.

### Inhibition of Cerebral Inflammation and Treatment Indications

Multiple sclerosis (MS) is a chronic demyelinating disease that affects neurons in the CNS, resulting in a range of debilitating physical and mental symptoms. Disease progression is characterized by increasing disability, and life expectancy is lowered by 7-14 years. MS affects an estimated 2.3 million worldwide, including 400,000 people in the United States. The pathogenesis of MS involves immune attack against self-antigens in the CNS, mediated through activated CD4+ myelin-reactive T lymphocytes, with a contribution by B lymphocytes. It is still unclear how selftolerance is broken, but once this is achieved, myelin-reactive T cells migrate across the blood-brain barrier in a process involving interaction between adhesion molecules present on T lymphocytes and capillary endothelial cells, facilitated by the local production of chemokines and matrix metalloproteinases. After entering the CNS, autoreactive T lymphocytes are reactivated by recognizing autoantigenic peptides presented by local antigen presenting cells (dendritic cells, macrophages, and B lymphocytes), triggering an inflammatory cascade involving further release of cytokines and chemokines and further recruitment and persistent activation of leukocytes, resulting in chronic myelin damage.

The present inventors created an EAE model that generates both spinal cord and cerebral inflammation, thus closely resembling human MS pathology. The present inventors further discovered that accompanying cerebral inflammation in this EAE model, there is a strong increase in CCL5/RANTES production in the brain, which may be caused by clustering of CCLS-expressing brain resident memory T cells.

These findings provide a novel cellular and molecular link between CCL5/RANTES and cerebral inflammation, thus forming the basis for using a CCR5 inhibitor to inhibit cerebral inflammation in a subject in need thereof. As cerebral inflammation is implicated in MS and a number of other neuroinflammatory disorders, CCR5 inhibitors that inhibit cerebral inflammation can also be used for treating and/or preventing these disorders.

"Treating" or "treatment of", or "preventing" or "prevention of", as used herein, refers to an approach for obtaining beneficial or desired results. Beneficial or desired results can include, but are not limited to, alleviation or amelioration of one or more symptoms or conditions, diminishment of extent of disease, stabilisation of the state of disease, prevention of development of disease, prevention of spread of disease, delay or slowing of disease progression (e.g. suppression), delay or slowing of disease onset, conferring protective immunity against a disease-causing agent and amelioration or palliation of the disease state. "Treating" or "preventing" can also mean prolonging survival of a patient beyond that expected in the absence of treatment and can also mean inhibiting the progression of disease temporarily or preventing the occurrence of disease, such as by preventing infection in a subject.

"Treating" may be distinguished from "preventing" in that "treating" typically occurs in a subject who already has a disease or disorder, or is known to have already been exposed to an infectious agent, whereas "preventing" typically occurs in a subject who does not have a disease or disorder, or is not known to have been exposed to an infectious agent. As will be appreciated, there may be overlap in treatment and prevention. For example, it is possible to be "treating" a disease in a subject, while at same time "preventing" symptoms or progression of the disease.

As used herein, an "individual" or a "subject" is a mammal, for example, a human. Mammals also include, but are not limited to, farm animals, sport animals, pets, primates, and horses.

In some embodiments, the subject suffers from or is susceptible to a neuroinflammatory disorder that involves cerebral inflammation. Such neuroinflammatory disorders include, but are not limited to, Alzheimer's Disease, Parkinson's Disease, multiple sclerosis, viral encephalitis, Rasmussen encephalitis, progressive multifocal leukoencephalopathy (PML) including PML-associated immune reconstitution inflammatory syndrome (IRIS), cerebral malaria, HIV-associated neurocognitive disorders, CNS vasculitis, antibody-mediated inflammatory brain disorders, and neurosarcoidosis.

In some embodiments, the subject suffers from or is susceptible to multiple sclerosis, Rasmussen encephalitis, progressive multifocal leukoencephalopathy (PML) including PML-associated immune reconstitution inflammatory syndrome (IRIS), cerebral malaria or HIV-associated neurocognitive disorders.

In some embodiments, the subject suffers from or is susceptible to multiple sclerosis.

As used herein, the phrase "multiple sclerosis" refers to the inflammatory, demyelinating disease of the central nervous system (CNS) which is typically characterized by various symptoms of neurological dysfunction. Any type of MS may be treated according to the teachings of the present invention including relapsing-remitting, secondary progressive, primary progressive, progressive relapsing and special cases of MS with non-standard behavior (also referred to as borderline forms of MS), such as for example without limitation, tumefactive MS, neuromyelitis optica (NMO), Balo concentric sclerosis, Schilder disease, Marburg multiple sclerosis, and acute disseminated encephalomyelitis (ADEM).

The most common type of MS is relapsing-remitting MS (RRMS) where periods of stability (remission) are followed by episodes when there are exacerbations of symptoms (relapses). About 85% of people with MS have RRMS at onset. Two thirds of these go on to develop secondary progressive MS, where relapses are associated with progressively less complete recovery, and eventually worsening symptoms develop without any clear remissions. About 15% of people with MS have primary progressive MS (PPMS), where symptoms gradually develop and worsen over time without periods of relapse and remission.

Alzheimer's disease (AD) is the most common age-associated neurodegenerative disorder. Neuroinflammation is an important contributor to the pathogenesis of AD. Numerous studies show that microglial-mediated inflammation contributes to the progression of AD and that microglial cells are found in close association with amyloid-β (Aβ) deposits. It is likely that in the AD brain some activated microglial species may, in certain circumstances, adopt a proinflammatory profile with deleterious effects, promoting neuronal and synaptic damage. Several immunological mediators, including chemokines and cytokines, are elevated in the brain and cerebrospinal fluid of patients with AD. In particular, elevated expression of RANTES has been shown in the cerebral microcirculation of AD patients.

Parkinson's disease (PD) is characterized by the loss of dopaminergic (DA) neurons and the presence of α-synuclein-containing aggregates in the substantia nigra pars compacta (SNpc). Chronic neuro inflammation is one of the hallmarks of PD pathophysiology. Post-mortem analyses of human PD patients and experimental animal studies indicate that activation of glial cells and increases in pro-inflammatory factor levels are common features of the PD brain. Chronic release of proinflammatory cytokines by activated astrocytes and microglia leads to the exacerbation of DA neuron degeneration in the SNpc. Levels of the chemokine RANTES associated with recruitment of inflammatory cells have been observed to be elevated in patients with PD.

Encephalitis is an inflammation of the brain. It is most frequently caused by viral infection, including infection by herpes simplex virus types 1 and 2, Japanese encephalitis virus, enteroviruses such as poliovirus and coxsackievirus, rabies virus, tick-borne arboviruses such as that causing Powassan encephalitis, or mosquito-transmitted viruses such as those causing West Nile, La Crosse, St. Louis, western equine and eastern equine encephalitis. Neuronal injury in encephalitis patients is attributed to a number of proinflammatory cytokines, and upregulation of RANTES gene expression in neuroglia by Japanese encephalitis virus infection has been reported.

Rasmussen encephalitis (RE) is a chronic pediatric disease characterized by unihemispheric lymphocytic infiltrates, microglial nodules and neuronal destruction that is responsible for drug-resistant focal epilepsy and progressive neurological and cognitive deterioration. Cerebral hemispherectomy remains the only treatment for the associated seizures even though it inevitably results in functional impairment. Histopathological studies have shown involvement of clonally expanded cytotoxic CD8+ T cells and activated microglia and astroglia. CD8+ T cells that infiltrate the brain in RE express CCR5.

Immune reconstitution inflammatory syndrome (IRIS) is a condition seen in some cases of acquired immune deficiency syndrome (AIDS) or immunosuppression, in which the immune system begins to recover, but then responds to a previously acquired opportunistic infection with an overwhelming inflammatory response that paradoxically makes the symptoms of infection worse. The vast majority of CD8+ T cells that infiltrate inflammatory CNS lesions resulting from progressive multifocal leukoencephalopathy (PML) express high levels of CCR4.

With more than 300 million new cases and about two million deaths annually, malaria represents the most important worldwide parasitic disease. The most severe complication of this disease is cerebral malaria (CM), which involves the cytoadherence of parasitized erythrocytes (PEs) to the cerebral microvasculature. Infection of susceptible mouse strains with Plasmodium berghei ANKA (PbA) provides an experimental model of CM that shares some characteristics with the human disease. CCR5 expression on both brain-sequestered leukocytes and nonhematopoietic cells, including possibly endothelial cells, astrocytes, and microglial cells in the brain, is required for CM to develop in the PbA-infected mice.

HIV-associated neurocognitive disorder (HAND) is a term used to describe the spectrum of neurocognitive dysfunction associated with HIV infection. It results from the direct neurotoxicity of HIV and from consequences of the host immune response. For example, infection causes parenchymal release of pro-inflammatory cytokines and chemokines from infected cells, leading to neuronal degeneration and death. CCR5 inhibitors may inhibit this vicious cycle by limiting recruitment of leukocytes to the CNS and local inflammation mediated by CCRS+ astrocytes and microglial cells.

CNS vasculitis is the inflammation of blood vessel walls in the brain, resulting in restricted blood flow. It is the most common inflammatory brain disease in children. It is also known as cerebral vasculitis, isolated angiitis of the CNS (IACNS), transient cerebral arteriopathy (TCA), or transient cerebral vasculopathy; childhood CNS vasculitis may be referred to as childhood primary angiitis of the CNS (cPACNS). It is currently treated with immunosuppressants.

In antibody-mediated inflammatory disease, B cells produce autoantibodies against the body's own structures. When autoantibodies bind to these structures, they induce inflammation that is directed against healthy tissue. Autoantibodies directed against structures in the brain lead to irritation and swelling of brain tissue. If not treated, long standing inflammation can lead to permanent brain damage and dysfunction. Currently known antibody-mediated inflammatory brain disorders include anti-N-methyl-D-aspartate receptor (NMDAR) encephalitis, limbic encephalitis, Hashimoto encephalitis, and pediatric autoimmune neuropsychiatric disorders associated with streptococcal infections (PANDAS). These diseases are usually treated with immunosuppressants.

Neurosarcoidosis refers to sarcoidosis, a chronic inflammatory disorder characterized by abnormal cell deposits known as granulomas, that manifests in any part of the nervous system. It most commonly occurs in the cranial and facial nerves, the hypothalamus, and the pituitary gland. It is estimated to develop in 5 to 15 percent of those individuals who have sarcoidosis. Its cause is unknown.

A neuroinflammatory disorder that involves cerebral inflammation may be treated according to the methods of the present invention at any stage including, but not limited to, when the subject is undergoing an acute attack. According to some embodiments, the disorder may be treated chronically for preventing outbreaks and the damage caused therefrom, during flare-ups of the disorder for reducing and preventing deterioration or both. According to some embodiments, a CCR5 inhibitor, pharmaceutical composition or dosage form provided herein is administered before the outbreak, or appearance of symptoms, of the disorder. According to some embodiments, the disorder is treated in patients that are resistant and/or not sensitive to conventional treatments, such as treatments with steroids. According to some embodiments, the symptoms of the disorder are treated by a CCR5 inhibitor, pharmaceutical composition or dosage form provided herein.

CCR5 inhibitors, pharmaceutical compositions and dosage forms provided herein may be administered by any means that achieve their intended purpose. For example, administration may be by carried out orally, sublingually, buccally, topically, rectally, via inhalation, transdermally, subcutaneously, intravenously, intra-arterially or intramuscularly, via intracardiac administration, intraosseously, intradermally, intraperitoneally, intracranially, intracerebrally, transmucosally, vaginally, intravitreally, epicutaneously, intra-articularly, intravesically, intrathecally, periarticularly or locally. The dosage administered will be dependent upon the age, health, and weight of the subject, kind of concurrent treatment, if any, frequency of treatment, and the nature of the effect desired.

According to some embodiments, a CCR5 inhibitor provided herein may be administered orally, possibly in the form of tablets, capsules, powders, troches, soft gelatin capsules, syrup, liquid suspension or lozenges. According to some embodiments, a CCR5 inhibitor provided herein may be administered in the form of a parenteral formulation, such as for subcutaneous intramuscular or intravenous administration. According to further embodiments, a CCR5 inhibitor provided herein may be administered by any appropriate nasal administration, pulmonary administration, topically or any appropriate dermatological administration.

According to some embodiments, a CCR5 inhibitor provided herein may be administered together with any appropriate non-toxic pharmaceutical carrier. The carrier may be a gas, solid or liquid. According to some embodiments, the carrier is selected from saline solution, water, any appropriate emulsion or dispersion or any combination thereof.

According to some embodiments, a CCR5 inhibitor provided herein may be administered together with any ingredients appropriate for modifying the release of the CCR5 inhibitor from the dosage form comprising the CCR5 inhibitor. For example, time delaying agents, such as enteric coated gelatin capsules, may be used.

According to some embodiments, in methods of treating MS, a CCR5 inhibitor, pharmaceutical composition or dosage form provided herein may be administered together with any additional active ingredients that are known to have therapeutic efficacy against MS, such as without being limited, corticosteroids (e.g., methylprednisolone, prednisone, prednisolone, dexamethasone), adrenocorticotrophic hormone (ACTH), beta-interferons (e.g., interferon-β1a, interferon-β1b, interferon-β2), interferon-τ, mitoxantrone (Novantrone^{®}), natalizumab (Tysabri^{®}), fingolimod (Gilenya^{®}), glatiramer acetate (Copaxone^{®}), teriflunomide (Aubagio^{®}), ocrelizumab (Ocrevus^{®}), dimethyl fumarate (Tecfidera^{®}), alemtuzumab (Lemtrada^{®}), spirogermaniums (e.g., *N*-(3-dimethylaminopropyl)-2-aza-8,8-dimethyl-8-germaspiro [4:5] decane, *N*-(3 -dimethylaminopropyl)-2-aza-8,8-diethyl-8 - germaspiro [4:5] decane, *N*-(3 -dimethylaminopropyl)-2-aza-8,8-dibutyl-8 - germaspiro[4:5]decane), vitamin D analogs, prostaglandins (e.g., latanoprost, brimonidine, PGE1, PGE2 and PGE3), tetracyclines (e.g., minocycline and doxycycline), 2-chlorodeoxyadenosine, sulphasalazine, methotrexate, azathioprine, cyclophosphamide, cyclosporine, tizanidine hydrochloride, or any appropriate combination thereof.

The amount of a CCR5 inhibitor provided herein administered according to the methods of the present invention will vary, as recognized by those skilled in the art, dependent on route of administration, excipient usage, and the possibility of co-usage with other therapeutic treatments including use of other therapeutic agents. According to some embodiments, a CCR5 inhibitor described herein may be administered in an amount of from about 0.1 mg to about 400 mg. According to some embodiments, a CCR5 inhibitor provided herein may be administered in an amount of from about 1 mg to about 100 mg. According to some embodiments, a CCR5 inhibitor provided herein may be administered in an amount of from about 4 mg to about 40 mg.

According to some embodiments, a CCR5 inhibitor provided herein may be administered in an amount of from about 0.1 mg to about 300 mg. According to some embodiments, a CCR5 inhibitor provided herein may be administered in an amount of from about 0.1 mg to about 200 mg. According to some embodiments, a CCR5 inhibitor provided herein may be administered in an amount of from about 0.1 mg to about 100 mg. According to some embodiments, a CCR5 inhibitor provided herein may be administered in an amount of from about 0.1 mg to about 95 mg. According to some embodiments, a CCR5 inhibitor provided herein may be administered in an amount of from about 0.1 mg to about 90 mg. According to some embodiments, a CCR5 inhibitor provided herein may be administered in an amount of from about 0.1 mg to about 85 mg. According to some embodiments, a CCR5 inhibitor provided herein may be administered in an amount of from about 0.1 mg to about 80 mg. According to some embodiments, a CCR5 inhibitor provided herein may be administered in an amount of from about 0.1 mg to about 75 mg. According to some embodiments, a CCR5 inhibitor provided herein may be administered in an amount of from about 0.1 mg to about 70 mg. According to some embodiments, a CCR5 inhibitor provided herein may be administered in an amount of from about 0.1 mg to about 65 mg. According to some embodiments, a CCR5 inhibitor provided herein may be administered in an amount of from about 0.1 mg to about 60 mg. According to some embodiments, a CCR5 inhibitor provided herein may be administered in an amount of from about 0.1 mg to about 55 mg. According to some embodiments, a CCR5 inhibitor provided herein may be administered in an amount of from about 0.1 mg to about 50 mg. According to some embodiments, a CCR5 inhibitor provided herein may be administered in an amount of from about 0.1 mg to about 45 mg. According to some embodiments, a CCR5 inhibitor provided herein may be administered in an amount of from about 0.1 mg to about 40 mg. According to some embodiments, a CCR5 inhibitor provided herein may be administered in an amount of from about 0.1 mg to about 35 mg. According to some embodiments, a CCR5 inhibitor provided herein may be administered in an amount of from about 0.1 mg to about 30 mg. According to some embodiments, a CCR5 inhibitor provided herein may be administered in an amount of from about 0.1 mg to about 25 mg. According to some embodiments, a CCR5 inhibitor provided herein may be administered in an amount of from about 0.1 mg to about 20 mg. According to some embodiments, a CCR5 inhibitor provided herein may be administered in an amount of from about 0.1 mg to about 15 mg. According to some embodiments, a CCR5 inhibitor provided herein may be administered in an amount of from about 0.1 mg to about 10 mg. According to some embodiments, a CCR5 inhibitor provided herein may be administered in an amount of from about 0.1 mg to about 5 mg. According to some embodiments, a CCR5 inhibitor provided herein may be administered in an amount of from about 0.1 mg to about 1 mg.

According to some embodiments, a CCR5 inhibitor provided herein may be administered in an amount of from about 1 mg to about 400 mg. According to some embodiments, a CCR5 inhibitor provided herein may be administered in an amount of from about 1 mg to about 300 mg. According to some embodiments, a CCR5 inhibitor provided herein may be administered in an amount of from about 1 mg to about 200 mg. According to some embodiments, a CCR5 inhibitor provided herein may be administered in an amount of from about 1 mg to about 95 mg. According to some embodiments, a CCR5 inhibitor provided herein may be administered in an amount of from about 1 mg to about 90 mg. According to some embodiments, a CCR5 inhibitor provided herein may be administered in an amount of from about 1 mg to about 85 mg. According to some embodiments, a CCR5 inhibitor provided herein may be administered in an amount of from about 1 mg to about 80 mg. According to some embodiments, a CCR5 inhibitor provided herein may be administered in an amount of from about 1 mg to about 75 mg. According to some embodiments, a CCR5 inhibitor provided herein may be administered in an amount of from about 1 mg to about 70 mg. According to some embodiments, a CCR5 inhibitor provided herein may be administered in an amount of from about 1 mg to about 65 mg. According to some embodiments, a CCR5 inhibitor provided herein may be administered in an amount of from about 1 mg to about 60 mg. According to some embodiments, a CCR5 inhibitor provided herein may be administered in an amount of from about 1 mg to about 55 mg. According to some embodiments, a CCR5 inhibitor provided herein may be administered in an amount of from about 1 mg to about 50 mg. According to some embodiments, a CCR5 inhibitor provided herein may be administered in an amount of from about 1 mg to about 45 mg. According to some embodiments, a CCR5 inhibitor provided herein may be administered in an amount of from about 1 mg to about 40 mg. According to some embodiments, a CCR5 inhibitor provided herein may be administered in an amount of from about 1 mg to about 35 mg. According to some embodiments, a CCR5 inhibitor provided herein may be administered in an amount of from about 1 mg to about 30 mg. According to some embodiments, a CCR5 inhibitor provided herein may be administered in an amount of from about 1 mg to about 25 mg. According to some embodiments, a CCR5 inhibitor provided herein may be administered in an amount of from about 1 mg to about 20 mg. According to some embodiments, a CCR5 inhibitor provided herein may be administered in an amount of from about 1 mg to about 15 mg. According to some embodiments, a CCR5 inhibitor provided herein may be administered in an amount of from about 1 mg to about 10 mg. According to some embodiments, a CCR5 inhibitor described herein may be administered in an amount of from about 1 mg to about 5 mg.

According to some embodiments, a CCR5 inhibitor provided herein may be administered in an amount of from about 5 mg to about 400 mg. According to some embodiments, a CCR5 inhibitor provided herein may be administered in an amount of from about 5 mg to about 300 mg. According to some embodiments, a CCR5 inhibitor provided herein may be administered in an amount of from about 5 mg to about 200 mg. According to some embodiments, a CCR5 inhibitor provided herein may be administered in an amount of from about 5 mg to about 100 mg. According to some embodiments, a CCR5 inhibitor provided herein may be administered in an amount of from about 5 mg to about 95 mg. According to some embodiments, a CCR5 inhibitor provided herein may be administered in an amount of from about 5 mg to about 90 mg. According to some embodiments, a CCR5 inhibitor provided herein may be administered in an amount of from about 5 mg to about 85 mg. According to some embodiments, a CCR5 inhibitor provided herein may be administered in an amount of from about 5 mg to about 80 mg. According to some embodiments, a CCR5 inhibitor provided herein may be administered in an amount of from about 5 mg to about 75 mg. According to some embodiments, a CCR5 inhibitor provided herein may be administered in an amount of from about 5 mg to about 70 mg. According to some embodiments, a CCR5 inhibitor provided herein may be administered in an amount of from about 5 mg to about 65 mg. According to some embodiments, a CCR5 inhibitor provided herein may be administered in an amount of from about 5 mg to about 60 mg. According to some embodiments, a CCR5 inhibitor provided herein may be administered in an amount of from about 5 mg to about 55 mg. According to some embodiments, a CCR5 inhibitor provided herein may be administered in an amount of from about 5 mg to about 50 mg. According to some embodiments, a CCR5 inhibitor provided herein may be administered in an amount of from about 5 mg to about 45 mg. According to some embodiments, a CCR5 inhibitor provided herein may be administered in an amount of from about 5 mg to about 40 mg. According to some embodiments, a CCR5 inhibitor provided herein may be administered in an amount of from about 5 mg to about 35 mg. According to some embodiments, a CCR5 inhibitor provided herein may be administered in an amount of from about 5 mg to about 30 mg. According to some embodiments, a CCR5 inhibitor provided herein may be administered in an amount of from about 5 mg to about 25 mg. According to some embodiments, a CCR5 inhibitor provided herein may be administered in an amount of from about 5 mg to about 20 mg. According to some embodiments, a CCR5 inhibitor provided herein may be administered in an amount of from about 5 mg to about 15 mg. According to some embodiments, a CCR5 inhibitor provided herein may be administered in an amount of from about 5 mg to about 10 mg.

According to some embodiments, a CCR5 inhibitor provided herein may be administered in an amount of from about 10 mg to about 400 mg. According to some embodiments, a CCR5 inhibitor provided herein may be administered in an amount of from about 10 mg to about 300 mg. According to some embodiments, a CCR5 inhibitor provided herein may be administered in an amount of from about 10 mg to about 200 mg. According to some embodiments, a CCR5 inhibitor provided herein may be administered in an amount of from about 10 mg to about 100 mg. According to some embodiments, a CCR5 inhibitor provided herein may be administered in an amount of from about 10 mg to about 95 mg. According to some embodiments, a CCR5 inhibitor provided herein may be administered in an amount of from about 10 mg to about 90 mg. According to some embodiments, a CCR5 inhibitor provided herein may be administered in an amount of from about 10 mg to about 85 mg. According to some embodiments, a CCR5 inhibitor provided herein may be administered in an amount of from about 10 mg to about 80 mg. According to some embodiments, a CCR5 inhibitor provided herein may be administered in an amount of from about 10 mg to about 75 mg. According to some embodiments, a CCR5 inhibitor provided herein may be administered in an amount of from about 10 mg to about 70 mg. According to some embodiments, a CCR5 inhibitor provided herein may be administered in an amount of from about 10 to about 65 mg. According to some embodiments, a CCR5 inhibitor provided herein may be administered in an amount of from about 10 mg to about 60 mg. According to some embodiments, a CCR5 inhibitor provided herein may be administered in an amount of from about 10 mg to about 55 mg. According to some embodiments, a CCR5 inhibitor provided herein may be administered in an amount of from about 10 mg to about 50 mg. According to some embodiments, a CCR5 inhibitor provided herein may be administered in an amount of from about 10 mg to about 45 mg. According to some embodiments, a CCR5 inhibitor provided herein may be administered in an amount of from about 10 mg to about 40 mg. According to some embodiments, a CCR5 inhibitor provided herein may be administered in an amount of from about 10 mg to about 35 mg. According to some embodiments, a CCR5 inhibitor provided herein may be administered in an amount of from about 10 mg to about 30 mg. According to some embodiments, a CCR5 inhibitor provided herein may be administered in an amount of from about 10 mg to about 25 mg. According to some embodiments, a CCR5 inhibitor provided herein may be administered in an amount of from about 10 mg to about 20 mg. According to some embodiments, a CCR5 inhibitor provided herein may be administered in an amount of from about 10 mg to about 15 mg.

According to some embodiments, a CCR5 inhibitor provided herein may be administered in an amount of about 400 mg, about 350 mg, about 300 mg, about 250 mg, about 200 mg, about 150 mg, about 100 mg, about 95 mg, about 90 mg, about 85 mg, about 80 mg, about 75 mg, about 70 mg, about 65 mg, about 60 mg, about 55 mg, about 50 mg, about 45 mg, about 40 mg, about 35 mg, about 30 mg, about 25 mg, about 20 mg, about 19 mg, about 18 mg, about 17 mg, about 16 mg, about 15 mg, about 14 mg, about 13 mg, about 12 mg, about 11 mg, about 10 mg, about 9 mg, about 8 mg, about 7 mg, about 6 mg, about 5 mg, about 4 mg, about 3 mg, about 2 mg, about 1 mg, about 0.5 mg, or about 0.1 mg.

According to some embodiments, a CCR5 inhibitor, pharmaceutical composition or dosage form provided herein may be administered once a day or in separate administrations of 2, 3, 4, 5 or 6 equal doses per day. According to other embodiments, a CCR5 inhibitor, pharmaceutical composition or dosage form provided herein may be administered twice, thrice, four times, five times, six times per day, or more. According to some embodiments, a CCR5 inhibitor, pharmaceutical composition or dosage form provided herein may be administered chronically. According to some embodiments, a CCR5 inhibitor, pharmaceutical composition, or dosage form provided herein may be administered for one, two, three, four, five, six, or seven days; for one, two, three, or four weeks; for one, two, three, four, five, six, seven, eight, nine, ten, eleven, twelve months or longer.

For chronical administration, a CCR5 inhibitor, pharmaceutical composition or dosage form provided herein may be administered at a frequency of daily, six times a week, five times a week, four times a week, three times a week, twice a week, once a week, once every two weeks, once every three weeks, once every four weeks, once every five weeks, once every six weeks, once every seven weeks, once every eight weeks, once every nine weeks, once every ten weeks, twice a month, once a month, once every two months, or once every three months.

In some embodiments, a CCR5 inhibitor, pharmaceutical composition or dosage form provided herein may be administered or provided for administration separately, sequentially or simultaneously in combination with a further pharmacologically active compound. In some embodiments, a CCR5 inhibitor, pharmaceutical composition or dosage form provided herein is used in conjunction with a further pharmacologically active compound. Alternatively, the therapeutic administration of a CCR5 inhibitor, pharmaceutical composition or dosage form provided herein may precede or follow the further pharmacologically active compound treatment by intervals ranging from minutes to weeks. In embodiments where the CCR5 inhibitor, pharmaceutical composition or dosage form provided herein and the further pharmacologically active compound are administered separately, one would generally ensure that a significant period of time did not expire between each delivery, such that the CCR5 inhibitor, pharmaceutical composition or dosage form provided herein and the pharmacologically active compound would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, where several days (2, 3, 4, 5, 6 or 7) to several weeks (1, 2, 3, 4, 5, 6, 7 or 8) lapse between the respective administrations.

A CCR5 inhibitor, pharmaceutical composition, or dosage form described herein may be administered until the subject in need thereof does not require treatment, prophylaxis, or amelioration of any disorder or condition such as, for example, multiple sclerosis.

It should be understood, however, that a specific dosage and treatment regimen for any particular subject will depend upon a variety of factors, including the activity of the specific compound employed, the age, body weight, general health, sex, diet, time of administration, rate of excretion, drug combination, and the judgment of the treating physician and the severity of the particular disorder being treated. The amount of a CCR5 inhibitor can also depend upon the therapeutic or prophylactic agent, if any, with which the CCR5 inhibitor is co-administered.

### EXAMPLES

### Example 1: Transient postnatal brain virus infection precipitates neuro inflammation at sites of previous infection

Intracranial (i.c.) infection with the attenuated strain of lymphocytic choriomeningitis virus rLCMV/INDG (hereafter referred to as rLCMV) is an established model to study transient brain infection in adult C57BL/6 mice. This model was extended by performing rLCMV i.c. infection in young mice to reproduce childhood infection (Figure 1a). I.c. administration of rLCMV in 1-week-old and > 3-week-old mice induced similar expansion of virus-specific antiviral CD8+ T cells (Figure 1b), and in both experimental settings, virus levels were reduced below the detection limit of quantitative real-time PCR within 5 weeks after infection (Figure 1c).

In order to investigate if such transient virus infection may impact neuroinflammation later in life, in vitro activated 2D2 TCR-transgenic MOG-specific CD4+ T cells were transferred intraperitoneally (i.p.) into virus-cleared mice or age-matched mock-infected controls (Figure 2a, Figure 1d).

Regardless of age during viral infection, mice that received 2D2 T cells developed classical symptoms of experimental autoimmune encephalomyelitis (EAE), namely, ascending paralysis, to a similar extent as their age-matched mock-infected controls (Figure 1e, f). Measurement of the classical EAE score was done by observation of classical symptoms on a scale of 0 to 5 (0: disease-free, 1: tail paralysis, 2: hind limb paresis, 3: hind limb paralysis, 4: hind limb paralysis and forelimb paresis, 5: moribund or dead). The classical symptoms were associated with comparable 2D2 T cell expansion in the blood as well as recruitment and development of autoimmune lesions in the spinal cord (Figure 1g-j).

However, mice infected at 1 week of age showed an increased incidence (Figure 2b) and severity (Figure 2c) of atypical symptoms (namely, ataxia and proprioception deficits) in comparison to age-matched mock-infected controls, which was not observed in mice infected at > 3 weeks of age (Figure 2d, e). Measurement of the atypical EAE score was done by observation of atypical symptoms on a scale of 0 to 5 (0: disease-free, 1: scrubby fur, hunching, 2: ataxia, 3: temporary proprioception deficits (impaired balance, leaning to one side), 4: continuous and severe proprioception deficits (constant falling on one side without the possibility to regain proper body alignment), 5: moribund or death). Immunohistochemical analysis detected the presence of autoimmune lesions containing 2D2 T cells (CD45.1) in the cerebra of mice infected at 1 week of age (Figure 2f), and a significantly increased recruitment of 2D2 T cells to this area was observed in comparison to mice infected at > 3 weeks of age (Figure 2g). Accordingly, numbers of brain lesions were significantly higher in mice previously infected at 1 week of age as compared to control animals (Figure 2h), while mice previously infected at > 3w of age did not show signs of increased brain inflammation (Figure 2i).

Next, the spatial association between brain areas that were previously exposed to viral infection and the occurrence of cerebral EAE lesions was examined. To trace virus spread, 1-week-old or > 3-week-old LoxP-flanked RFP reporter mice (St-RFP) were infected with rLCMV expressing Cre recombinase (rLCMV-Cre) (Figure 3a). rLCMV-Cre infection irreversibly induces reporter gene expression in virus-experienced cells. As observed for viral RNA (compare Figure 1), no viral protein (Figure 3b) could be detected 5 weeks after infection. Yet, RFP-expressing (RFP+) virus-purged reporter cells were still detectable around the brain ventricles and in the corpus callosum in adult mice infected at 1 week of age, but not in mice infected at > 3 weeks of age (Figure 3b, c). This is consistent with a partial non-cytolytic virus clearance mechanism after postnatal rLCMV infection, while in older mice, infected cells were all eliminated. Immunofluorescent co-staining of RFP+ cells in reporter mice infected with rLCMV-Cre at 1 week of age marked virus spread beyond ependymal linings to parenchymal neurons and glial cells (Figure 3d).

Following 2D2 T cell transfer into these mice (Figure 4a), the majority (75%) of cerebral EAE lesions localized in direct contact or in proximity to virus-purged RFP+ reporter cells (Figure 4b). Virus-experienced RFP+ cells could represent a potential source of persisting virus antigen despite undetectable virus levels. Therefore, whether these cells are essential for the development of observed EAE lesions in the cerebrum was tested.

To this end, Cre recombinase-inducible diphtheria toxin receptor (iDTR) mice were infected with rLCMV-Cre at 1 week of age and depleted virus-experienced cells by administration of diphtheria toxin (DT) three weeks later (Figure 4c). rLCMV-Cre infection in St-RFP-iDTR double transgenic mice confirmed that DT administration eliminated >95% of virus-experienced cells (Figure 5a, b). The development of classical EAE and spinal cord inflammation was not altered by DT administration (Figure 5c-e). Moreover, depletion of virus-experienced cells did neither protect mice from development of atypical symptoms nor did it reduce the number of cerebral lesions (Figure 4d-f), suggesting that the existence of virus-experienced cells alone is not sufficient to explain the observed clinical and histopathological EAE phenotype in mice previously infected at 1 week of age.

### Example 2: Transient postnatal brain virus leaves a persisting pro--chemotactic environment

Given the spatial but non-causal relationship of cerebral EAE lesions and virus-experienced cells, the inflammatory tissue microenvironment of RFP+ reporter cells was next characterized. The expression of inflammation-related genes in tissue punches from brain areas containing RFP+ reporter cells was compared with equivalent areas from adult mice infected > 3 weeks of age and mock-infected control animals (Figure 6a). While virus-purged mice infected at > 3 weeks of age grouped together with mock-infected controls, adult mice that had been infected at 1 week of age showed a differential gene expression pattern (Figure 6b). The latter revealed persistent expression of several pro-inflammatory genes, with highest induction of CCL5/RANTES as compared to a pool of both control groups (Figure 6c).

Flow cytometric analysis identified brain resident memory T cells (bT_{RM}) as main source of CCL5 in both experimental groups (58 ± 2% or 55 ± 2%, respectively) (Figure 6d), while CD4+ T cells, monocyte-derived cells or microglia expressed only minute amounts (data not shown). bT_{RM} obtained from adult mice infected at 1 week of age had a comparable phenotype to bT_{RM} obtained from adult mice infected at > 3 weeks of age with respect to bona fide bT_{RM} markers CD69 and Granzyme B11 (Figure 7a-c), but were more numerous (Figure 7d), leading to a significantly increased number of CCL5+ bT_{RM} in adult mice infected at 1 week of age compared to mice infected at > 3 weeks of age (Figure 6e). In contrast to bT_{RM}, only a small proportion of splenic memory T cells (T_{M}) expressed CCL5 protein (Figure 7e, f), and numbers of splenic CCL5+ T_{M} were similar in mice previously infected at 1 week or > 3 weeks of age (Figure 7g).

Immunohistochemical analysis confirmed bT_{RM} as major source of CCL5, with 81 ± 4.1% CCL5+ cells co-expressing CD8 (Figure 6f). While bT_{RM} were observed in both previously-infected experimental groups, clustering of these cells was a distinguishing feature for mice infected at 1 week of age and only very rarely observed in mice infected > 3 weeks of age (Figure 6g). This suggests that CCL5+ bT_{RM} clustering in higher density in the surrounding of RFP+ reporter cells is responsible for the observed strong increase in local CCL5 production.

### Example 3: 5P12-RANTES treatment

In an embodiment of the CCR5 inhibitor for use according to the claimed invention, mice were treated with 5P12-RANTES (SEQ ID NO: 70) after transfer of 2D2 T cells (Figure 6h). Treatment with 5P12-RANTES abrogated the development of atypical EAE symptoms (Figure 6i, j) and significantly reduced the number of brain lesions to levels of mock--infected controls (Figure 6k). Moreover, treatment with 5P12-RANTES provided stronger protection than Met-RANTES (intraperitoneally administered at 10 µg per day for 2 weeks), not only in terms of reduced infiltration of inflammatory cells into the sites of previous infection, but also significant reduction in neuroinflammation pathology (data not shown).

The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

It is to be understood that any numerical value inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

It must be noted that as used in this specification and the appended claims, the singular forms "a," "an," and "the" include plural reference unless the context clearly dictates otherwise. Unless defined otherwise all technical and scientific terms used herein have the same meaning as commonly understood to one of ordinary skill in the art to which this invention belongs.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to encompass the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items.

As used herein, whether in the specification or the appended claims, the transitional terms "comprising", "including", "carrying", "having", "containing", "involving", and the like are to be understood as being inclusive or open-ended (i.e., to mean including but not limited to), and they do not exclude unrecited elements, materials or method steps. Only the transitional phrases "consisting of" and "consisting essentially of", respectively, are closed or semi-closed transitional phrases with respect to claims and exemplary embodiment paragraphs herein. The transitional phrase "consisting of" excludes any element, step, or ingredient which is not specifically recited. The transitional phrase "consisting essentially of" limits the scope to the specified elements, materials or steps and to those that do not materially affect the basic characteristic(s) of the invention disclosed and/or claimed herein.

## Claims

1. A CCR5 inhibitor for use in treating a neuroinflammatory disorder that involves cerebral inflammation in a subject, wherein the CCR5 inhibitor is a polypeptide comprising an N-terminal portion and a C-terminal portion, wherein the N-terminal portion comprises the signature sequence QGP[P or L] and the amino acid sequence of the C-terminal portion is at least 70% identical to SEQ ID NO: 1.

2. The CCR5 inhibitor for use according to claim 1, wherein the CCR5 inhibitor leads to a signaling response of 30% or less of the maximum response (Emax) elicited by PSC-RANTES, when tested at a concentration of 300 nM in a Calcium Flux signaling assay.

3. The CCR5 inhibitor for use according to claim 2, wherein the CCR5 inhibitor is selective for CCR5 over CCR1 and CCR3.

4. The CCR5 inhibitor for use according to claim 1, wherein the signature sequence is QGP[P or L] [L or G or S or M] [M or D or S or Q or G].

5. The CCR5 inhibitor for use according to claim 1, wherein the signature sequence is QGP[P or L][L or G or S or M][M or D or S or Q or G]XX[Q or G or L or A or T or S]X, wherein X denotes any natural or modified amino acid.

6. The CCR5 inhibitor for use according to claim 1, wherein the signature sequence is QGP[P or L]LM or QGPPG[D or S].

7. The CCR5 inhibitor for use according to claim 1, wherein the signature sequence is QGPPLM or QGPPGD.

8. The CCR5 inhibitor for use according to claim 1, wherein the signature sequence is QGP[P or L][L or M][M or Q][A or W or G or Q or N]X[Q or G or L][S or V or T or G], wherein X denotes any natural or modified amino acid.

9. The CCR5 inhibitor for use according to claim 1, wherein the signature sequence is QGPPLM[A or W][L or T or M][Q or G][S or V or T or G].

10. The CCR5 inhibitor for use according to claim 1, wherein the signature sequence is QGPP[G or L][M or Q]XX[Q or S][S or V], wherein X denotes any natural or modified amino acid.

11. The CCR5 inhibitor for use according to claim 1, wherein the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) and QGPPMQGGLS (SEQ ID NO: 43).

12. The CCR5 inhibitor for use according to claim 1, wherein the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) and QGPPLMQTTP (SEQ ID NO: 15).

13. The CCR5 inhibitor for use according to claim 1, wherein the signature sequence is QGPPLMATQS (SEQ ID NO: 9).

14. The CCR5 inhibitor for use according to any one of claims 1 to 13, wherein the C-terminal portion is identical to SEQ ID NO: 1.

15. The CCR5 inhibitor for use according to claim 1, wherein the polypeptide comprises the amino acid sequence of SEQ ID NO: 70.

16. The CCR5 inhibitor for use according to any one of claims 1 to 15, wherein the neuroinflammatory disorder is Alzheimer's Disease, Parkinson's Disease, multiple sclerosis, viral encephalitis, Rasmussen encephalitis, progressive multifocal leukoencephalopathy (PML) including PML-associated immune reconstitution inflammatory syndrome (IRIS), cerebral malaria, HIV-associated neurocognitive disorders, CNS vasculitis, antibody-mediated inflammatory brain disorders, or neurosarcoidosis.

17. The CCR5 inhibitor for use according to claim 16, wherein the neuroinflammatory disease is multiple sclerosis, Rasmussen's encephalitis, progressive multifocal leukoencephalopathy (PML) including PML-associated immune reconstitution inflammatory syndrome (IRIS), cerebral malaria or HIV-associated neurocognitive disorders.

18. The CCR5 inhibitor for use according to claim 17, wherein the neuroinflammatory disease is multiple sclerosis.

## Patentansprüche

1. CCRS-Inhibitor zur Verwendung bei der Behandlung einer neuroinflammatorischen Störung, die eine zerebrale Entzündung in einem Patienten beinhaltet, wobei der CCR5-Inhibitor ein Polypeptid ist, das einen N-terminalen Abschnitt und einen C-terminalen Abschnitt umfasst, wobei der N-terminale Abschnitt die Signatursequenz QGP[P oder L] umfasst und die Aminosäuresequenz des C-terminalen Abschnitts zu mindestens 70 % identisch mit SEQ ID NO: 1 ist.

2. Der CCRS-Inhibitor zur Verwendung nach Anspruch 1, wobei der CCRS-Inhibitor zu einer Signalantwort von 30 % oder weniger der durch PSC-RANTES ausgelösten maximalen Antwort (Emax) führt, wenn er bei einer Konzentration von 300 nM in einem Calciumfluss-Signaltest getestet wird.

3. Der CCRS-Inhibitor zur Verwendung nach Anspruch 2, wobei der CCRS-Inhibitor selektiv für CCR5 gegenüber CCR1 und CCR3 ist.

4. Der CCRS-Inhibitor zur Verwendung nach Anspruch 1, wobei die Signatursequenz QGP[P oder L] [L oder G oder S oder M] [M oder D oder S oder Q oder G] ist.

5. Der CCRS-Inhibitor zur Verwendung nach Anspruch 1, wobei die Signatursequenz QGP[P oder L][L oder G oder S oder M][M oder D oder S oder Q oder G]XX[Q oder G oder L oder A oder T oder S]X ist, wobei X eine beliebige natürliche oder modifizierte Aminosäure bezeichnet.

6. Der CCRS-Inhibitor zur Verwendung nach Anspruch 1, wobei die Signatursequenz QGP[P oder L]LM oder QGPPG[D oder S] ist.

7. Der CCRS-Inhibitor zur Verwendung nach Anspruch 1, wobei die Signatursequenz QGPPLM oder QGPPGD ist.

8. Der CCRS-Inhibitor zur Verwendung nach Anspruch 1, wobei die Signatursequenz QGP[P oder L][L oder M][M oder Q][A oder W oder G oder Q oder N]X[Q oder G oder L][S oder V oder T oder G] ist, wobei X eine beliebige natürliche oder modifizierte Aminosäure bezeichnet.

9. Der CCRS-Inhibitor zur Verwendung nach Anspruch 1, wobei die Signatursequenz QGPPLM[A oder W][L oder T oder M] [Q oder G] [S oder V oder T oder G] ist.

10. Der CCRS-Inhibitor zur Verwendung nach Anspruch 1, wobei die Signatursequenz QGPP[G oder L][M oder Q]XX[Q oder S][S oder V] ist, wobei X eine beliebige natürliche oder modifizierte Aminosäure bezeichnet.

11. Der CCRS-Inhibitor zur Verwendung nach Anspruch 1, wobei die Signatursequenz ausgewählt ist aus der Gruppe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) und QGPPMQGGLS (SEQ ID NO: 43).

12. Der CCRS-Inhibitor zur Verwendung nach Anspruch 1, wobei die Signatursequenz ausgewählt ist aus der Gruppe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) und QGPPLMQTTP (SEQ ID NO: 15).

13. Der CCRS-Inhibitor zur Verwendung nach Anspruch 1, wobei die Signatursequenz QGPPLMATQS (SEQ ID NO: 9) ist.

14. Der CCRS-Inhibitor zur Verwendung nach einem der Ansprüche 1 bis 13, wobei der C-terminale Abschnitt mit SEQ ID NO: 1 identisch ist.

15. Der CCRS-Inhibitor zur Verwendung nach Anspruch 1, wobei das Polypeptid die Aminosäuresequenz von SEQ ID NO: 70 umfasst.

16. Der CCRS-Inhibitor zur Verwendung nach einem der Ansprüche 1 bis 15, wobei die neuroinflammatorische Störung Alzheimer, Parkinson, Multiple Sklerose, virale Enzephalitis, Rasmussen-Enzephalitis, progressive multifokale Leukoenzephalopathie (PML), einschließlich PML-assoziiertem Immunrekonstitutionsentzündungssyndrom (IRIS), zerebrale Malaria, HIV-assoziierte neurokognitive Störung, ZNS-Vaskulitis, Antikörper-vermittelte entzündliche Gehirnstörungen oder Neurosarkoidose ist.

17. Der CCRS-Inhibitor zur Verwendung nach Anspruch 16, wobei die neuroinflammatorische Erkrankung Multiple Sklerose, Rasmussen-Enzephalitis, progressive multifokale Leukoenzephalopathie (PML) einschließlich des PMLassoziierten entzündlichen Immunrekonstitutionssyndroms (IRIS), zerebrale Malaria oder HIV-assoziierte neurokognitive Störungen ist.

18. Der CCRS-Inhibitor zur Verwendung nach Anspruch 17, wobei die neuroinflammatorische Erkrankung Multiple Sklerose ist.

## Revendications

1. Un inhibiteur de CCR5 pour utilisation dans le traitement d'un trouble neuroinflammatoire impliquant une inflammation cérébrale chez un sujet, dans lequel l'inhibiteur de CCR5 est un polypeptide comprenant une partie N-terminale et une partie C-terminale, où la partie N-terminale comprend la séquence de signature QGP[P ou L] et la séquence d'acides aminés de la partie C-terminale est identique à au moins 70 % à SEQ ID NO : 1.

2. L'inhibiteur de CCR5 pour l'utilisation selon la revendication 1, dans lequel l'inhibiteur de CCR5 entraîne une réponse de signalisation de 30 % ou moins de la réponse maximale (Emax) provoquée par PSC-RANTES, lorsqu'il est testé à une concentration de 300 nM dans un essai de signalisation du flux calcique.

3. L'inhibiteur de CCR5 pour l'utilisation selon la revendication 2, dans lequel l'inhibiteur de CCR5 est sélectif pour le CCR5 par rapport à CCR1 et à CCR3.

4. L'inhibiteur de CCR5 pour l'utilisation selon la revendication 1, dans lequel la séquence de signature est QGP[P ou L] [L ou G ou S ou M] [M ou D ou S ou Q ou G].

5. L'inhibiteur de CCR5 pour l'utilisation selon la revendication 1, dans lequel la séquence de signature est QGP[P ou L][L ou G ou S ou M][M ou D ou S ou Q ou G]XX[Q ou G ou L ou A ou T ou S]X, où X désigne tout acide aminé naturel ou modifié.

6. L'inhibiteur de CCR5 pour l'utilisation selon la revendication 1, dans lequel la séquence de signature est QGP[P ou L]LM ou QGPPG[D ou S].

7. L'inhibiteur de CCR5 pour l'utilisation selon la revendication 1, dans lequel la séquence de signature est QGPPLM ou QGPPGD.

8. L'inhibiteur de CCR5 pour l'utilisation selon la revendication 1, dans lequel la séquence de signature est QGP[P ou L][L ou M][M ou Q][A ou W ou G ou Q ou N]X[Q ou G ou L][S ou V ou T ou G], où X désigne tout acide aminé naturel ou modifié.

9. L'inhibiteur de CCR5 pour l'utilisation selon la revendication 1, dans lequel la séquence de signature est QGPPLM[A ou W][L ou T ou M][Q ou G][S ou V ou T ou G].

10. L'inhibiteur de CCR5 pour l'utilisation selon la revendication 1, dans lequel la séquence de signature est QGPP[G ou L][M ou Q]XX[Q ou S][S ou V], où X désigne tout acide aminé naturel ou modifié.

11. L'inhibiteur de CCR5 pour l'utilisation selon la revendication 1, dans lequel la séquence de signature est sélectionnée dans le groupe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) and QGPPMQGGLS (SEQ ID NO: 43).

12. L'inhibiteur de CCR5 pour l'utilisation selon la revendication 1, dans lequel la séquence de signature est sélectionnée dans le groupe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) and QGPPLMQTTP (SEQ ID NO: 15).

13. L'inhibiteur de CCR5 pour l'utilisation selon la revendication 1, dans lequel la séquence de signature est QGPPLMATQS (SEQ ID NO: 9).

14. L'inhibiteur de CCR5 pour l'utilisation selon une quelconque des revendications 1 à 13, dans lequel la partie C-terminale est identique à SEQ ID NO : 1.

15. L'inhibiteur de CCR5 pour l'utilisation selon la revendication 1, dans lequel le polypeptide comprend la séquence d'acides aminés de SEQ ID NO : 70.

16. L'inhibiteur de CCR5 pour l'utilisation selon une quelconque des revendications 1 à 15, dans lequel le trouble neuroinflammatoire est la maladie d'Alzheimer, la maladie de Parkinson, la sclérose en plaques, l'encéphalite virale, l'encéphalite de Rasmussen, la leucoencéphalopathie multifocale progressive (LMP), y compris le syndrome inflammatoire de reconstitution immunitaire (IRIS) associé à la LMP, la malaria cérébrale, les troubles neurocognitifs associés au VIH, la vascularite du SNC, les troubles cérébraux inflammatoires à médiation par les anticorps ou la neurosarcoïdose.

17. L'inhibiteur de CCR5 pour l'utilisation selon la revendication 16, dans lequel la maladie neuroinflammatoire est la sclérose en plaques, l'encéphalite de Rasmussen, la leucoencéphalopathie multifocale progressive (LMP), y compris le syndrome inflammatoire de reconstitution immunitaire (IRIS) associé à la LMP, la malaria cérébrale ou les troubles neurocognitifs associés au VIH.

18. L'inhibiteur de CCR5 pour l'utilisation selon la revendication 17, dans lequel la maladie neuroinflammatoire est la sclérose en plaques.
